# EUROPEAN PATENT APPLICATION

(11) **EP 1 138 693 A1**
(43) Date of publication of application: **04.10.2001**
(21) Application number: 99961285.6
(22) Date of filing: 09.12.1999
(51) Int. Cl.: C07K 14/705, C12N 15/12, C12P 21/02, C07K 16/28, A61K 39/395, G01N 33/53

(54) **NOVEL G PROTEIN-COUPLED RECEPTOR PROTEIN AND DNA THEREOF**

(30) Priority: 11.12.1998 JP 35316598; 08.02.1999 JP 2967799
(71) Applicant: Takeda Chemical Industries, Ltd., Osaka-shi, Osaka 541-0045 (JP)
(72) Inventor: WATANABE, Takuya, Osaka-shi Osaka 532-0033 (JP); KIKUCHI, Kuniko, Toride-shi Ibaraki 302-0024 (JP); SHINTANI, Yasushi, Tsukuba-shi Ibaraki 305-0821 (JP)
(74) Representative: Keller, Günter, Dr.
(86) International application number: JP9906904
(87) International publication number: WO0035953

(57) **Abstract**

The G protein coupled receptor protein of the present invention, a partial peptide thereof or a salt thereof, as well as a polynucleotide encoding the same (for example, DNA, RNA and a derivative thereof), is useful in (1) determination of a ligand (agonist), (2) acquisition of an antibody and antiserum thereto, (3) construction of an expression system for a recombinant receptor protein thereof, (4) development of a binding assay system using said expression system and screening of a pharmaceutical candidate compound, (5) practice of a drug design based on comparison with structurally analogous ligand receptors, (6) reagents for preparation of probes and PCR primers in gene diagnosis, (7) preparation of a transgenic animals or (8) a pharmaceutical composition for a preventing and treating genetic agent etc.

## Description

### Technical Field

The present invention relates to a mouse spleen-derived or human lung-derived novel G protein coupled receptor protein or a salt thereof and a DNA encoding the same.

### Background Art

Physiologically active substances such as many hormones and neurotransmitters regulate the function of the living body through specific receptor proteins present in a cell membrane. Since many of these receptor proteins conduct intracellular signal transduction through activation of coupled guanine nucleotide-binding protein (hereinafter abbreviated as G protein in some cases) and have the common structure having 7 transmembrane regions, they are called collectively as G protein coupled receptor protein or 7 times transmembrane type receptor protein (7 TMR).

The G protein coupled receptor protein is present on the surfaces of the cells and various functional cells of organs of the living body and plays a physiologically important role as a target for a molecule which regulates the functions of those cells and organs, for example, hormones, neurotransmitters and physiologically active substances. A receptor transmits a signal into a cell through binding with a physiologically active substance, and a variety of reactions such as activation and inhibition of a cell are induced by this signal.

Elucidation of the relationship of a substance which regulates the complex function of cells and organs of a variety of living bodies and a specific receptor protein, in particular, a G protein coupled receptor protein elucidates the function of cells and organs of a variety of living bodies, and provides a very important means for developing drugs which are closely associated with the functions.

For example, in a variety of organs of the living body, the physiological regulation is conducted under regulation by many hormones, hormone-like substances, neurotransmitters or physiologically active substances. In particular, physiologically active substances are present in various sites in vivo, and regulate their physiological function through receptor proteins, each corresponding thereto. There are still unknown hormones and neurotransmitters and other physiologically active substances in vivo, many of which have not been reported yet regarding the structures of these receptor proteins. Further, whether or not a subtype is present is not known in many of the known receptor proteins.

Revelation of the relationship between a substance which regulates the complex function in vivo and a specific receptor protein therefor is a very important means for developing pharmaceutical compositions. In addition, in order to effectively screen an agonist and an antagonist for receptor proteins and develop pharmaceutical compositions, it is necessary to elucidate the function of a gene of a receptor protein expressed in vivo and express it in a suitable expression system.

Recently, as a means for analyzing a gene expressed in vivo, studies of analyzing randomly a sequence of a cDNA are actively conducted, and the thus obtained sequences of cDNA fragments are registered in database as Expressed Sequence Tag (EST) and published. However, many ESTs contain only sequence information and it is difficult to presume their functions.

Previously, a substance which inhibits binding of a G protein coupled receptor and a physiologically active substance (that is, ligand) and a substance which causes the same signal transduction as that for a physiologically active substance (that is, ligand) by binding have been utilized as a pharmaceutical composition for regulating the functions of the living body as a specific antagonist or agonist for these receptors. Therefore, new finding of a G protein coupled receptor protein which is not only important in the physiological manifestation in vivo but also as a target for drug development, and cloning of the gene (for example, cDNA) are a very important means upon finding of a specific ligand, an agonist and an antagonist for a novel G protein coupled receptor protein.

However, not all of G protein coupled receptors have been found and, even at present, there are still many unknown G protein coupled receptors, and so called orphan receptors for which corresponding ligands have not been identified, and there are desired search of new G protein coupled receptors and elucidation of their functions.

A G protein coupled receptor is useful for searching a new physiologically active substance (that is, ligand) using its signal transduction action as an index, or searching an agonist or antagonist for the receptor. On the other hand, even when a physiological ligand has not been found, an agonist or an antagonist to the receptor can be prepared by analyzing the physiological action of the receptor from an inactivation experiment of the receptor (knockout animal). A ligand, an agonist or an antagonist for these receptors can be expected to be utilized as an agent for preventing/treating or diagnosing diseases associated with the dysfunction of a G protein coupled receptor.

Further, there are many cases where reduction or sthenia of the function of the receptor in vivo based on a gene mutation of a G protein coupled receptor is a cause for some diseases. In this case, there can be applied to gene therapy by not only administration of an antagonist or an agonist for the receptor but also introduction of the receptor gene into the living body (or a particular organ) and introduction of an antisense nucleic acid for the receptor gene. In this case, a base sequence of the receptor is essential information for examining the presence or absence of deletion or mutation on a gene and the receptor gene can be applied to an agent for preventing/treating or diagnosing diseases associated with the dysfunction of the receptor.

The present invention provides a novel G protein coupled receptor protein which is useful as described above. That is, the present invention provides a novel G protein coupled receptor protein or a partial peptide thereof or a salt thereof, a polynucleotide (DNA, RNA and their derivatives) comprising a polynucleotide (DNA, RNA and their derivatives) encoding the G protein coupled receptor protein or a partial peptide thereof, a recombinant vector comprising the polynucleotide, a transformant harboring the recombinant vector, a process for producing the G protein coupled receptor protein or a salt thereof, an antibody to the G protein coupled receptor protein or a partial peptide thereof or a salt thereof, a compound which alters an amount of the G protein coupled receptor protein to be expressed, a method for determining a ligand for the G protein coupled receptor, a method for screening a compound (antagonist, agonist) or a salt thereof which alters binding a ligand with the G protein coupled receptor protein, a kit for the screening, a compound (antagonist, agonist) or a salt thereof which alters binding property of a ligand obtained by using the screening method or screening kit, with the G protein coupled receptor protein, and a pharmaceutical composition comprising a compound (antagonist, agonist) or a salt thereof which alters binding property of a ligand with the G protein coupled receptor protein and a compound which alters an amount of the G protein coupled receptor protein to be expressed.

### Disclosure of Invention

The present inventors studied intensively and, as a result, we isolated a cDNA encoding a mouse spleen-derived and human lung-derived novel G protein coupled receptor protein based on EST information made by a degenerated PCR method, and succeeded in analyzing its entire base sequence. When this base sequence was translated into an amino acid sequence, the first to seventh transmembrane regions were confirmed on hydrophobic plot, and proteins encoded by these cDNAs were confirmed to be 7 times transmembrane type G protein coupled receptor protein. The present inventors further studied on these findings, resulting in completion of the present invention.

That is, the present invention provides:
1. A G protein coupled receptor protein which comprises an amino acid sequence identical or substantially identical to an amino acid sequence represented by SEQ ID NO: 1 or a salt thereof .
2. The G protein coupled receptor protein according to the above 1, wherein the substantially identical amino acid sequence is an amino acid sequence represented by SEQ ID NO: 5.
3. A partial peptide of the G protein coupled receptor protein according to the above 1 or a salt thereof.
4. A polynucleotide which comprises a polynucleotide having a base sequence encoding the G protein coupled receptor protein according to the above 1.
5. The polynucleotide according to the above 4, which is a DNA.
6. The polynucleotide according to the above 4, which has a base sequence represented by SEQ ID NO: 2 or SEQ ID NO: 6.
7. A recombinant vector which comprises the polynucleotide according to the above 4.
8. A transformant transformed with the recombinant vector according to the above 7.
9. A method for manufacturing the G protein coupled receptor protein according to the above 1 or a salt thereof, which comprises culturing the transformant according to the above 8 to produce the G protein coupled receptor protein according to the above 1.
10. An antibody to the G protein coupled receptor protein according to the above 1 or the partial peptide according to the above 3 or a salt thereof.
11. The antibody according to the above 10, which is a neutralizing antibody, which inactivates signal transduction of the G protein coupled receptor protein according to the above 1.
12. A diagnostic drug, which comprises the antibody according to the above 10.
13. A ligand for the G protein coupled receptor protein according to the above 1 or a salt thereof, which is obtainable by using the G protein coupled receptor protein according to the above 1 or the partial peptide according to the above 3 or a salt thereof.
14. A pharmaceutical composition which comprises the ligand according to the above 13.
15. A method for determining a ligand for the G protein coupled receptor protein according to the above 1 or a salt thereof, which comprises using the G protein coupled receptor protein according to the above 1 or the partial peptide according to the above 3 or a salt thereof.
16. A method for screening a compound or a salt thereof which alters binding property of a ligand with the G protein coupled receptor protein or a salt thereof according to the above 1, which comprises using the G protein coupled receptor protein according to the above 1 or the partial peptide according to the above 3 or a salt thereof.
17. A kit for screening a compound or a salt thereof which alters binding property of a ligand with the G protein coupled receptor protein or a salt thereof according to the above 1, which comprises the G protein coupled receptor protein according to the above 1 or the partial peptide according to the above 3 or a salt thereof.
18. A compound or a salt thereof which alters binding property of a ligand with the G protein coupled receptor protein or a salt thereof according to the above 1, which is obtainable by using the method for screening according to the above 16 or the kit for screening according to the above 17.
19. A pharmaceutical composition which comprises a compound or a salt thereof which alters binding property of a ligand with the G protein coupled receptor protein or a salt thereof according to the above 1, which is obtainable by the method for screening according to the above 16 or the kit for screening according to the above 17.
20. A polynucleotide which hybridizes with the polynucleotide according to the above 4 under highly stringent conditions.
21. A polynucleotide which comprises a base sequence complementary to the polynucleotide according to the above 4 or a part thereof.
22. A method for quantifying an mRNA for the G protein coupled receptor protein according to the above 1, which comprises using the polynucleotide according to the above 4 or a part thereof.
23. A method for quantifying the G protein coupled receptor protein according to the above 1, which comprises using the antibody according to the above 10.
24. A diagnistic agent for diseases associated with the function of the G protein coupled receptor according to the above 1, which comprises using the quantifying method according to the above 22 or 23.
25. A method for screening a compound or a salt thereof which alters (enhances or suppresses) expression of the G protein coupled receptor according to the above 1 to be expressed, which comprises using the quantifying method according to the above 22 or 23.
26. A compound or a salt thereof which alters expression of the G protein coupled receptor protein according to the above 1, which is obtainable by the method for screening according to the above 25.

Further, the present invention provides:
27. The G protein coupled receptor protein or a salt thereof according to the above 1, wherein the protein is a protein comprising (a) an amino acid sequence represented by SEQ ID NO: 1, an amino acid sequence in which 1 or 2 or more (preferably about 1 to 30, morepreferably about 1 to 9, further more preferably 1 to 5) amino acids in an amino acid sequence represented by SEQ ID NO: 1 are deleted, (b) an amino acid sequence in which 1 or 2 or more (preferably about 1 to 30, more preferably about 1 to 10, further preferably 1 to 5) amino acids are added to an amino acid sequence represented by SEQ ID NO: 1, (c) an amino acid sequence in which 1 or 2 or more (preferably about 1 to 30, more preferably about 1 to 10, further preferably 1 to 5) amino acids in an amino acid sequence represented by SEQ ID NO: 1 are substituted with other amino acids, or (d) an amino acid of a combination of them.
28. A method for determining the ligand according to the above 15, which comprises contacting the G protein coupled receptor protein or a salt thereof according to the above 1 or the partial peptide or a salt thereof according to the above 3, with a test compound.
29. The method for determining a ligand according to the above 27, wherein the ligand is, for example, angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α- and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES), endothelin, enterogastrin, histamine, neurotensin, TRH, pancreatic polypeptide, galanin or LPA.
30. The method for screening according to the above 16, which comprises comparing (i) the case where the G protein coupled receptor protein or a salt thereof according to the above 1 or the partial peptide or a salt thereof according to the above 3 is contacted with a ligand, with (ii) the case where the G protein coupled receptor protein or a salt thereof according to the above 1 or the partial peptide or a salt thereof according to the above 3 is contacted with a ligand and a test compound.
31. A method for screening a compound or a salt thereof which alters binding property of a ligand with the G protein coupled receptor protein or a salt thereof according to the above 1, which comprises measuring and comparing an amount of a labeled ligand for the G protein coupled receptor protein or a salt thereof according to the above 1 or the partial peptide or a salt thereof according to the above 3 in (i) the case where a labeled ligand is contacted with the G protein coupled receptor protein or a salt thereof according to the above 1 or the partial peptide or a salt thereof according to the above 3, and (ii) the case where a labeled ligand and a test compound are contacted with the G protein coupled receptor protein or a salt thereof according to the above 1 or the partial peptide or a salt thereof according to the above 3.
32. A method for screening a compound or a salt thereof which alters binding property of a ligand with the G protein coupled receptor protein or a salt thereof according to the above 1, which comprises measuring and comparing an amount of a labeled ligand bound to a cell comprising the G protein coupled receptor protein according to the above 1 in (i) the case where a labeled ligand is contacted with the cell, and (ii) the case where a labeled ligand and a test compound are contacted with the cell.
33. A method for screening a compound or a salt thereof which alters binding property of a ligand with the G protein coupled receptor protein or a salt thereof according to the above 1, which comprises measuring and comparing an amount of a labeled ligand bound to a membrane fraction of a cell comprising the G protein coupled receptor protein according to the above 1 in (i) the case where a labeled ligand is contacted with the membrane fraction of the cell, and (ii) the case where a labeled ligand and a test compound are contacted with the membrane fraction of the cell.
34. A method for screening a compound or a salt thereof which alters binding property of a ligand with the G protein coupled receptor protein or a salt thereof according to the above 1, which comprises measuring and comparing an amount of a labeled ligand bound to the G protein coupled receptor protein in (i) the case where a labeled ligand is contacted with the G protein coupled receptor protein expressed on a cell membrane of the transformant according to the above 8 by culturing the transformant, and (ii) the case where a labeled ligand and a test compound are contacted with the G protein coupled receptor protein expressed on a cell membrane of the transformant according to the above 8 by culturing the transformant.
35. A method for screening a compound or a salt thereof which alters binding property of a ligand with the G protein coupled receptor protein or a salt thereof according to the above 1, which comprises measuring and comparing the cell stimulating activity via the G protein coupled receptor protein in (i) the case where a compound which activates the G protein coupled receptor protein or a salt thereof according to the above 1 is contacted with a cell comprising the G protein coupled receptor protein according to the above 1, and (ii) the case where a compound which activates the G protein coupled receptor protein or a salt thereof according to the above 1 and a test compound are contacted with a cell comprising the G protein coupled receptor protein according to the above 1.
36. A method for screening a compound or a salt thereof which alters binding property of a ligand with the G protein coupled receptor protein or a salt thereof according to the above 1, which comprises measuring and comparing the cell stimulating activity via the G protein coupled receptor protein in the case where a compound which activates the G protein coupled receptor protein or a salt thereof according to the above 1 is contacted with the G protein coupled receptor protein expressed on a cell membrane of the transformant according to the above 8 by culturing the transformant, and the case where a compound which activates the G protein coupled receptor protein or a salt thereof according to the above 1 and a test compound are contacted with the G protein coupled receptor protein expressed on a cell membrane of the transformant according to the above 8 by culturing the transformant.
37. The method for screening according to the above 34 or 36, wherein the compound which activates the G protein coupled receptor protein according to the above 1 is angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α- and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES), endothelin, enterogastrin, histamine, neurotensin, TRH, pancreatic polypeptide, galanin or LPA.
38. A compound or a salt thereof which alters binding property of a ligand with the G protein coupled receptor protein or a salt thereof according to the above 1, which is obtained by the method for screening according to the above 30 to 37.
39. A pharmaceutical composition which comprises a compound or a salt thereof which alters binding property of a ligand with the G protein coupled receptor protein or a salt thereof according to the above 1, which is obtained by the method for screening according to the above 30 to 37.
40. The kit for screening according to the above 17, which comprises a cell containing the G protein coupled receptor protein according to the above 1.
41. The kit for screening according to the above 17, which comprises a membrane fraction of a cell containing the G protein coupled receptor protein according to the above 1.
42. The kit for screening according to the above 17, which comprises a G protein coupled receptor protein expressed on a cell membrane of the transformant according to the above 8 by culturing said transformant.
43. A compound or a salt thereof which alters binding property of a ligand with the G protein coupled receptor protein or a salt thereof according to the above 1, which is obtained by using the kit for screening according to the above 40 to 42.
44. A pharmaceutical composition which comprises a compound or a salt thereof which alters binding property of a ligand with the G protein coupled receptor protein or a salt thereof according to the above 1, which is obtained by using the kit for screening according to the above 40 to 42.
45. A method for quantifying the G protein coupled receptor protein according to the above 1, the partial peptide according to the above 3 or a salt thereof, which comprises contacting the antibody according to the above 10 with the G protein coupled receptor protein according to the above 1, the partial peptide according to the above 2 or a salt thereof.
46. A method for quantifying the G protein coupled receptor protein according to the above 1, the partial peptide according to the above 3 or a salt thereof in a test solution, which comprises allowing a test solution and the labeled G protein coupled receptor protein according to the above 1, the partial peptide according to the above 3 or a salt thereof to react competitively with the antibody according to the above 10 and then measuring the ratio of the G protein coupled receptor protein according to the above 1, the partial peptide according to the above 3 or a salt thereof which is bound to said antibody.
47. A method for quantifying the G protein coupled receptor protein according to the above 1, the partial peptide according to the above 3 or a salt thereof in a test solution, which comprises allowing a test solution to react simultaneously or successively with the carrier-immobilized antibody according to the above 10 and the labeled antibody according to the above 10 and then measuring the activity of the labeling agent on the immobilizing carrier.

### Brief Description of Drawings

Fig. 1 shows a base sequence of a DNA encoding the novel mouse spleen-derived G protein coupled receptor protein mAL7T024 of the present invention obtained in Example 1, as well as an amino acid sequence deduced therefrom (continued to Fig. 2).
Fig. 2 shows a base sequence of a DNA encoding the novel mouse spleen-derived G protein coupled receptor protein mAL7T024 of the present invention obtained in Example 1, as well as an amino acid sequence deduced therefrom (continued from Fig. 1 to Fig. 3).
Fig. 3 shows a base sequence of a DNA encoding the novel mouse spleen-derived G protein coupled receptor protein mAL7T024 of the present invention obtained in Example 1, as well as an amino acid sequence deduced therefrom (continued from Fig. 2).
Fig. 4 shows a hydrophobic plot of the novel mouse spleen-derived G protein coupled receptor protein mAL7T024 of the present invention, which was made based on an amino acid sequence represented by Figs. 1 to 3.
Fig. 5 shows a base sequence of a DNA encoding the novel human lung-derived G protein coupled receptor protein hAL7T024 of the present invention obtained in Example 2, as well as an amino acid sequence deduced therefrom (continued to Fig. 6).
Fig. 6 shows a base sequence of a DNA encoding the novel human lung-derived G protein coupled receptor protein hAL7T024 of the present invention obtained in Example 2, as well as an amino acid sequence deduced therefrom (continued from Fig. 5 to Fig. 6).
Fig. 7 shows a base sequence of a DNA encoding the novel human lung-derived G protein coupled receptor protein hAL7T024 of the present invention obtained in Example 2, as well as an amino acid sequence deduced therefrom (continued to Fig. 6).
Fig. 8 shows a hydrophobic plot of the novel human lung-derived G protein coupled receptor protein hAL7T024 of the present invention, which was made based on an amino acid sequence represented by Figs. 5 to 7.

### Best Mode for Embodiment of the Invention

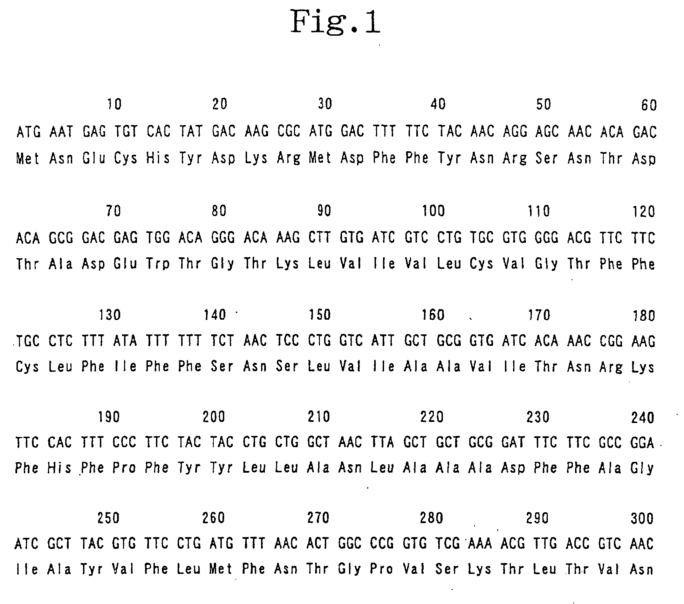

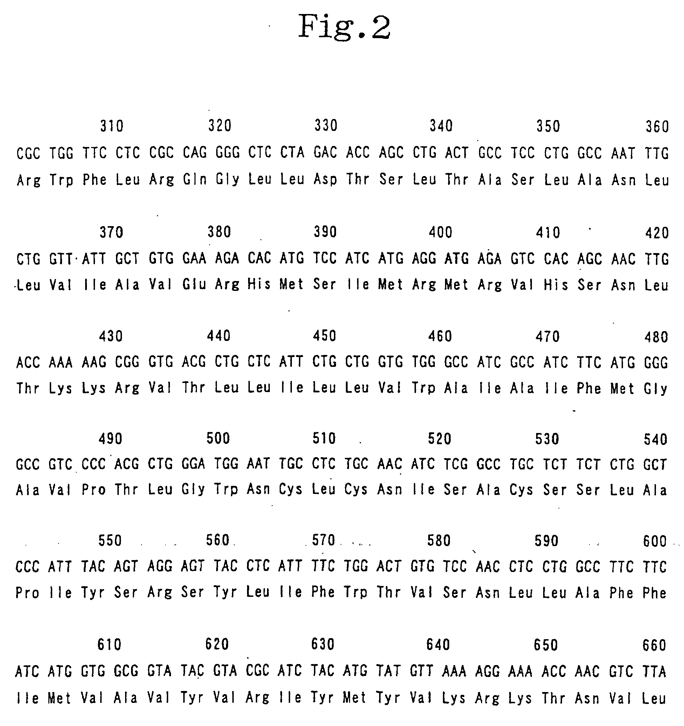

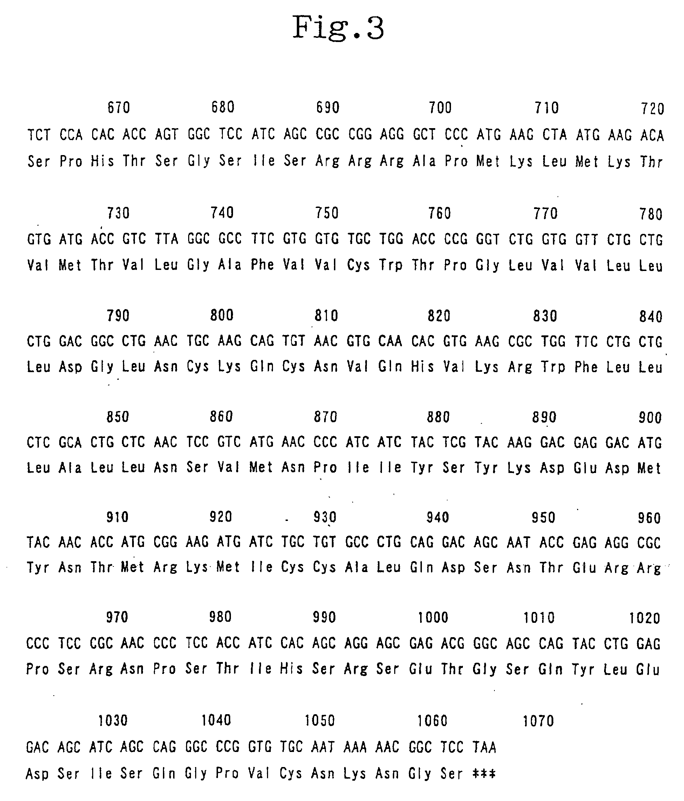

The present G protein coupled receptor protein (hereinafter abbreviated as receptor protein in some cases) is a receptor protein comprising an amino acid sequence identical or substantially identical to an amino acid sequence [amino acid sequence in Figs. 1 to 3] represented by SEQ ID NO: 1.

The present receptor protein may be a protein derived from cells (e.g., spleen cells, nerve cells, glia cells, pancreatic β-cells, bone marrow cells, mesangial cells, Langerhans' cells, epidermal cells, epithelial cells, endothelial cells, fibroblasts, fibrous cells, muscular cells, fat cells, immune cells (e.g., macrophage, T cell, B cell, natural killer cell, mast cell, neutrophils, basophils, eosinophils, monocyte), megakaryocyte, synovial cells, chondrocytes, bone cells, osteoblast, osteoclast, mammary gland cells, hepatic cells or interstitial cells, or their precursor cells, stem cells or cancer cells) , cells in the hemocyte system, or any tissues having such cells, for example, the brain and each site of brain (e.g., olfactory bulb, tonsil nuclei, cerebral basal bulb, hippocampus, thalamus, hypothalamus, hypothalamic nucleus, cerebral cortex, medulla bulb, cerebellum, occipital lobe, frontal lobe, temporal lobe, putamen, caudate nucleus, stained brain, substantia nigra), spinal cord, pituitary, stomach, pancreas, kidney, liver, gonads, thyroid glands, galls, bone marrow, adrenals, skin, muscles, lungs, digestive tracts (e.g., large and small intestines) , blood vessels, heart, thymus, spleen, salivary glands, peripheral blood, peripheral blood cell, prostate, testicles, spermary, ovary, placenta, uterus, bone, joints, skeletal muscles (particularly the brain and each site of brain) , from humans or mammals (e.g., guinea pig, rat, mouse, rabbit, pig, sheep, cow, monkey etc.), as well as a synthetic protein.

As an amino acid sequence substantially identical to an amino acid sequence represented by SEQ ID NO: 1, for example, an amino acid sequence having about 50% or more, preferably about 70% or more, more preferably about 80% or more, further preferably about 90% or more, and most preferably about 95% or more homology with an amino acid sequence represented by SEQ ID NO: 1, etc., are exemplified.

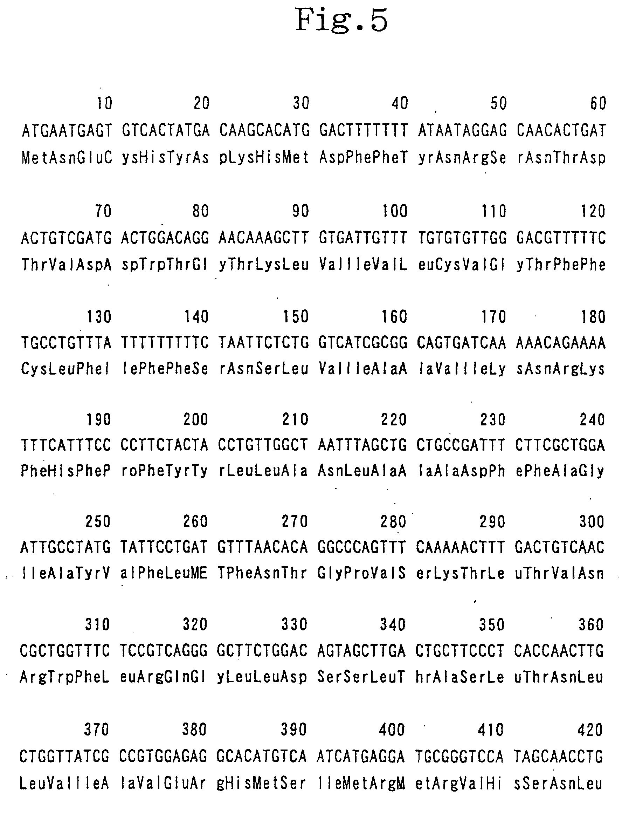

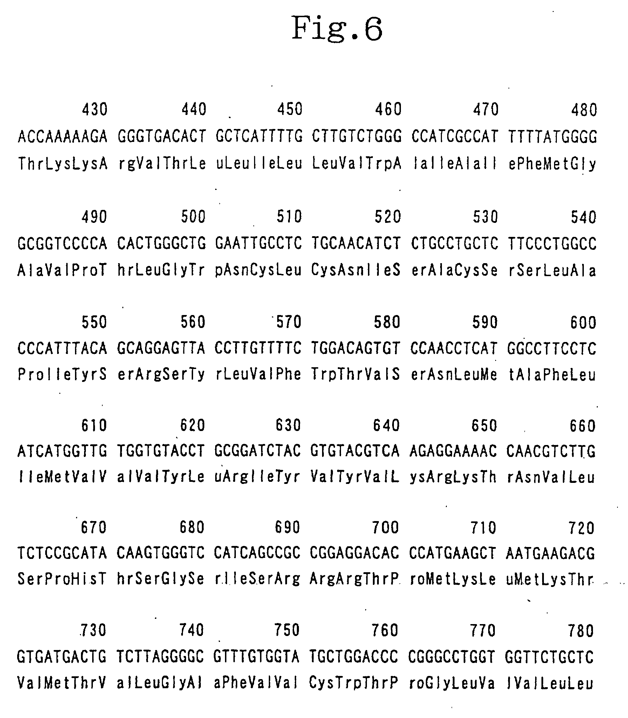

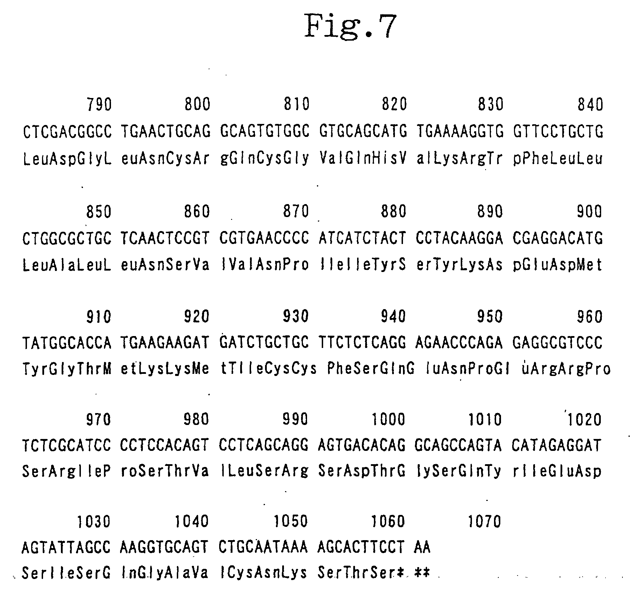

Specifically, a receptor protein comprising an amino acid sequence [amino acid sequence in Figs. 5 to 7] represented by SEQ ID NO: 5, etc., are exemplified.

The protein of the present invention comprising an amino acid sequence substantially identical to an amino acid sequence represented by SEQ ID NO: 1 is preferably, for example, a protein having an amino acid sequence substantially identical to the amino acid sequence represented by SEQ ID NO: 1 and having substantially homogenous activities to those of the amino acid sequence represented by SEQ ID NO: 1.

As the substantially homogenous activities, for example, ligand-binding activity, signal transduction action, etc. are exemplified. The terms "substantially homogenous" mean that these activities are qualitatively homogenous. Therefore, although it is preferable that the activities such as ligand-binding activity and signal transduction action are equal (e.g., about 0.01 - to 100-fold, preferably about 0.5 - to 20-fold, more preferably about 0.5- to 2-fold), quantitative elements such as a degree of these activities and the molecular weight of the protein may be different.

Measurement of the activities such as ligand-binding activity and signal transduction action can be carried out according to the publicly known method, for example, by the method for determining a ligand or the screening method as described later.

Further, as the receptor protein of the present invention, a protein comprising (1) an amino acid sequence in which 1 or 2 or more (preferably about 1 to 30, more preferably about 1 to 10, further preferably 1 to 5) amino acids in an amino acid sequence represented by SEQ ID NO: 1 are deleted, (2) an amino acid sequence in which 1 or 2 or more (preferably about 1 to 30, more preferably about 1 to 10, further preferably 1 to 5) amino acids are added to an amino acid sequence represented by SEQ ID NO: 1, (3) an amino acid sequence in which 1 or 2 or more (preferably about 1 to 30, more preferably about 1 to 10, further preferably 1 to 5) amino acids in an amino acid sequence represented by SEQ ID NO: 1 are substituted with other amino acids, or (4) a combination of these amino acid sequences, etc., are exemplified.

In addition, as the receptor protein of the present invention, a protein comprising (1) an amino acid sequence in which 1 or 2 or more (preferably about 1 to 30, more preferably about 1 to 10, further preferably 1 to 5) amino acids in an amino acid sequence represented by SEQ ID NO: 5 are deleted, (2) an amino acid sequence in which 1 or 2 or more (preferably about 1 to 30, more preferably about 1 to 10, further preferably 1 to 5) amino acids are added to an amino acid sequence represented by SEQ ID NO: 5, (3) an amino acid sequence in which 1 or 2 or more (preferably about 1 to 30, more preferably about 1 to 10, further preferably 1 to 5) amino acids in an amino acid sequence represented by SEQ ID NO: 5 are substituted with other amino acids, or (4) a combination of these amino acid sequences, etc., are exemplified.

In receptor proteins in the present specification, a left end is a N-terminal (amino terminal) and a right end is a C-terminal (carboxyl terminal) according to the convention of the peptide display. Although the receptor proteins of the present invention including a receptor protein comprising an amino acid sequence represented by SEQ ID NO: 1 have usually carboxyl group (-COOH) or carboxylate (-COO⁻) as a C-terminal, they may have an amide (-CONH₂) or an ester (-COOR) as a C- terminal.

As R in ester, for example C₁₋₆ alkyl group such as methyl, ethyl, n-propyl, isopropyl and n-butyl, C₃₋₈ cycloalkyl group such as cyclopentyl and cyclohexyl, C₆₋₁₂ aryl group such as phenyl and α-naphthyl, C₇₋₁₄ aralkyl group such as phenyl - C₁₋₂ alkyl group such as benzyl and phenetyl and α-naphthyl-C₁₋₂ alkyl group such as α-naphthylmethyl, as well as a pivaloyloxymethyl ester used generally in an oral ester are used.

When the receptor protein of the present invention has carboxyl group (or carboxylate) at a position other than a C-terminal, proteins in which carboxyl group is amidated or esterified are also included in the present receptor protein. As an ester used in this case, for example, an ester at a C-terminal described above is used.

Further, the present receptor protein includes proteins in which an amino group of a methionine residue of a N-terminal amino acid residue is protected with a protecting group (e.g., C₁₋₆ acyl group such as C₂₋₆ alkanoyl group such as formyl group, acetyl etc.) in the aforementioned proteins, proteins in which aN- terminal glutamyl group formed by cutting in vivo is converted into pyroglutamate, proteins in which a substituent group (e.g., -OH, -SH, amino group, imidazole group, indole group, guanidine group etc.) on a side chain of an intramolecular amino acid is protected with a suitable protecting group (e.g., C₁₋₆acyl group such as C₂₋₆ alkanoyl group such as formyl group, acetyl etc.), and conjugated proteins such as glycoprotein having sugar chains bound thereto.

As an embodiment of the present receptor protein, for example, a mouse-derived receptor protein comprising an amino acid sequence represented by SEQ ID NO: 1, a human-derived receptor protein comprising an amino acid sequence represented by SEQ ID NO: 5, etc. are used.

As a partial peptide of the present receptor protein (hereinafter abbreviated as partial peptide in some cases), any partial peptides are used as long as they are a partial peptide of the aforementioned receptor protein. For example, among the present receptor protein molecules, a partial peptide which is exposed outside a cell membrane and has the receptor binding activity is used.

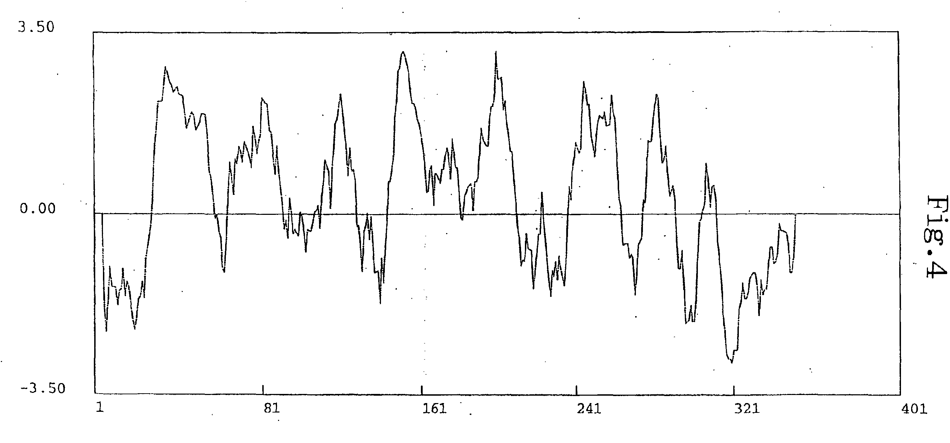

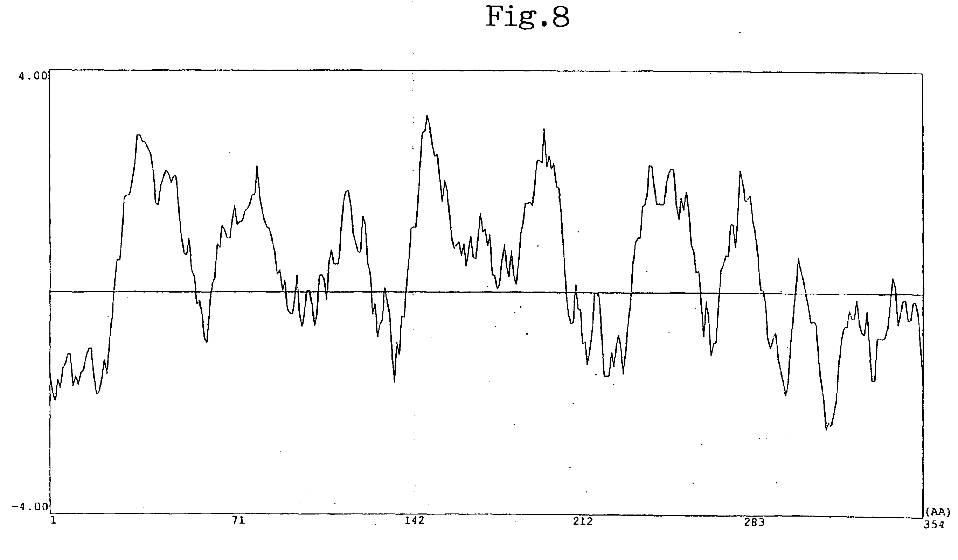

Specifically, as a partial peptide of a receptor protein having an amino acid sequence represented by SEQ ID NO: 1, there is a peptide comprising a part analyzed to be an extracellular region (hydrophilic site) in a hydrophobic plot analysis shown in Fig. 4. Further, as a partial peptide of a receptor protein having an amino acid sequence represented by SEQ ID NO: 5, there is a peptide comprising a part analyzed to be an extracellular region (hydrophilic site) in a hydrophobic plot analysis shown in Fig. 8. In addition, a peptide partially containing a hydrophobic site may also be used. Although peptides comprising individual domains separately may be used, peptides of a part comprising simultaneously a plurality of domains may be used.

It is preferable that the number of amino acids of the present partial peptide in the amino acid sequence of the aforementioned present receptor protein is at least 20 or more, preferably 50 or more, and more preferably 100 or more.

A substantially identical amino acid sequence denotes an amino acid sequence having about 70% or more, preferably about 80% or more, more preferably about 90% or more and further preferably about 95% or more homology with these amino acid sequences.

Here, the terms "substantially homogenous activities" have the same meanings as defined above. Measurement of "substantially homogeneous activities" can be performed as described above.

Further, in the present partial peptide, 1 or 2 or more (preferably about 1 to 10, more preferably 1 to 5) amino acids in the aforementioned amino acid sequence may be deleted, 1 or 2 or more (preferably about 1 to 20, more preferably about 1 to 10, further preferably 1 to 5) amino acids may be added to the amino acid sequence thereof, or 1 or 2 or more (preferably about 1 to 10, further preferably about 1 to 5) amino acids in the amino acid sequence thereof may be substituted with other amino acids.

Although the present partial peptide has usually carboxyl group (-COOH) or carboxylate (-COO⁻) as a C-terminal, it may have an amide (-CONH₂) or an ester (-COOR) as a C-terminal, as is the case with the present receptor protein described above.

Further, the present partial peptide, similar to the present receptor protein described above, includes peptides in which an amino group of a methionine residue as a N-terminal amino acid residue is protected with a protecting group, peptides in which a N-terminal Gln formed by cutting in vivo is converted into pyroglutamate, peptides in which a substituent group on a side chain of an intramolecular amino acid is protected with a suitable protecting group, and conjugated peptides such as glycopeptides having sugar chains bound thereto.

Although the present partial peptide has usually carboxyl group (-COOH) or carboxylate (-COO⁻) as a C-terminal, it may have an amide (-CONH₂) or an ester (-COOR) as a C-terminal, as with the present protein described above.

As a salt of the present receptor protein or a salt of a partial peptide thereof, physiologically acceptable acid addition salts are particularly preferable. As such salts, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid), etc. are used.

The present receptor protein or a salt thereof can be manufactured from cells or tissues of a human or a mammal described above by the publicly known method of purifying receptor proteins, or can be manufactured by culturing a transformant containing a DNA encoding the present receptor protein, as described below. Alternatively, it can also be manufactured by the protein synthesizing method described below or its modified method.

For manufacture from human or mammalian tissues or cells, the human or mammalian tissues or cells are homogenized and then extracted with e.g. an acid, and the extract can be subjected to purification and isolation by a combination of chromatographic techniques such as reverse-phase chromatography, ion-exchange chromatography etc.

For synthesis of the present receptor protein or a partial peptide thereof or a salt thereof or amide derivatives thereof, commercially available resin for protein synthesis can be usually used. Such resin includes, for example, chloromethyl resin, hydroxymethyl resin, benzhydryl amine resin, aminomethyl resin, 4-benzyloxybenzyl alcohol resin, 4-methylbenzhydryl amine resin, PAM resin, 4-hydroxymethyl methyl phenylacetamidemethyl resin, polyacrylamide resin, 4-(2',4'-dimethoxyphenyl-hydroxymethyl)phenoxy resin, 4-(2',4'-dimethoxyphenyl-Fmoc aminoethyl)phenoxy resin etc. On the resin described above, each amino acid with the α-amino group and side-chain functional group properly protected is condensed sequentially in accordance with the sequence of the desired protein by the publicly known condensation methods. At the end of the reaction, the protein is excised from the resin while various protecting groups are removed, and the product is subjected to a reaction of forming intramolecular disulfide bonds in a highly diluted solution to give the desired protein or an amide thereof.

A wide variety of activating reagents usable for protein synthesis can be used for condensation of the protected amino acids described above, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids along with racemization inhibitors (e.g., HOBt, HOOBt) can be added to the resin directly or after the protected amino acids were previously activated as corresponding acid anhydrides or HOBt esters or HOOBt esters.

The solvent used for activation of each protected amino acid or for condensation thereof with the resin can be selected as necessary from those solvents known to be usable in protein condensation reaction. Examples of such solvent include acid amides such as N,N-dimethylformamide, N,N-dimethylacetamide and N-methylpyrrolidone, halogenated hydrocarbons such as methylene chloride and chloroform, alcohols such as trifluoroethanol, sulfoxides such as dimethyl sulfoxide, ethers such as pyridine, dioxane and tetrahydrofuran, nitriles such as acetonitrile and propionitrile, esters such as methyl acetate and ethyl acetate, or a suitable mixture thereof. The reaction temperature is usually selected as necessary within the range known to be usable in the reaction of forming protein bonds, and usually the reaction temperature is selected within the range of about -20 °C to 50 °C. The activated amino acid derivatives are used usually in excess (1.5- to 4-fold). When their condensation is insufficient as a result of a test using ninhydrin reaction, their sufficient condensation is achieved by repeatedly carrying out the condensation reaction without conducting elimination of the protecting groups. When their sufficient condensation is not achieved even by repeatedly carrying out the reaction, the unreacted amino acids can be acetylated with acetic anhydride or acetyl imidazole.

The protecting groups for amino groups in the starting materials include, for example, Z, Boc, tert-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosfinothioyl, Fmoc etc.

The carboxyl group can be protected by, for example, alkyl esterification (e.g., straight-chain, branched or cyclic alkyl esterification such as methyl, ethyl, propyl, butyl, tert-butyl, cyclopentyl, cyclohexyl, cycloheptyl, cycloctyl or 2-adamantyl esterification), aralkyl esterification (e.g., benzyl esterification, 4-nitrobenzyl esterification, 4-methoxybenzyl esterification, 4-chlorobenzyl esterification, benzhydryl esterification), phenacyl esterification, benzyloxycarbonylhydrazidation, tert-butoxycarbonylhydrazidation, tritylhydrazidation etc.

The hydroxyl group in serine can be protected by, for example, esterification or etherification. A suitable group used in this esterification includes, for example, lower alkanoyl groups such as acetyl group, alloyl groups such as benzoyl group, and carbonic acid-derived groups such as benzyloxycarbonyl group and ethoxycarbonyl group. A suitable group for etherification includes, for example, a benzyl group, tetrahydropyranyl group, t-butyl group etc.

The protecting group used for the phenolic hydroxyl group in tyrosine includes, for example, Bzl, Cl₂-Bzl, 2-nitrobenzyl, Br-Z, tert-butyl etc.

The protecting group used for imidazole in histidine includes, for example, Tos, 4-methoxy-2,3,6-trimethylbenzenesulfonyl, DNP, benzyloxymethyl, Bum, Boc, Trt, Fmoc etc.

Activated carboxyl groups in the starting materials include, for example, the corresponding acid anhydrides, azides and active esters (i.e. esters with alcohols such as pentachlorophenol, 2,4,5-trichlorophenol, 2,4-dinitrophenol, cyanomethyl alcohol, p-nitrophenol, HONB, N-hydroxysuccinamide, N-hydroxyphthalimide and HOBt). The activated amino groups in the starting materials include, for example, the corresponding phosphoric acid amides.

Examples of methods for removing (leaving) the protecting groups include catalytic reduction in a hydrogen stream in the presence of a catalyst such as Pd-black or Pd-carbon, acid treatment with anhydrous hydrogen fluoride, methane sulfonic acid, trifluoromethane sulfonic acid, trifluoroacetic acid or a mixed solution thereof, base treatment using diisopropylethylamine, triethylamine, piperidine or piperazine, and reduction using sodium in liquid ammonia. The leaving reaction by the acid treatment described above is carried out generally at a temperature of about -20 °C to 40 °C, and it is effective in the acid treatment to add a cation scavenger such as anisole, phenol, thioanisole, m-cresol, p-cresol, dimethylsulfide, 1,4-butanedithiol and 1,2-ethanedithiol. A 2, 4-dinitrophenyl group used as a protecting group for imidazole in histidine can also be removed by treatment with thiophenol, while a formyl group used as a protecting group for indole in tryptophan can be removed not only by deprotection by acid treatment in the presence of 1,2-ethanedithiol or 1,4-butanedithiol, but also by alkali treatment using dilute sodium hydroxide solution or dilute ammonia.

Protection and protecting groups for functional groups which should not participate in the reaction of the starting materials, elimination of the protecting groups, and activation of functional groups participating in the reaction can be selected as necessary from known groups or known means.

Another method of obtaining an amide derivative of the protein includes, for example, protecting the α-carboxyl group of a C-terminal amino acid by amidation, then extending a peptide (protein) chain at the side of the amino group until it attains desired chain length, and thereafter manufacturing a protein of said peptide chain from which only the protecting group for the N-terminal α-amino group was removed and a protein of said peptide chain from which only the protecting group for the C- terminal carboxyl group was removed, followed by condensation both the proteins in the mixed solvent as described above. The details of the condensation reaction are the same as described above. The protected protein obtained by condensation is purified, and every protecting group is removed by the method descried above, whereby the desired crude protein can be obtained. This crude protein is purified by a variety of known purification techniques, and by lyophilizing its major fraction, the desired amide derivative of the protein can be obtained.

To obtain an ester derivative of the protein, for example the α-carboxyl group of a C-terminal amino acid is condensed with desired alcohol to form an amino acid ester from which the desired ester derivative of the protein can be obtained in the same manner as for the amide derivative of the protein.

The present receptor protein, a partial peptide thereof or a salt thereof can be manufactured according to a publicly known peptide synthesis method or by cleaving the present receptor protein with a suitable peptidase. For example, the peptide synthesis method may be the solid- or liquid-phase synthesis method. That is, the desired peptide can be manufactured by condensation of a partial peptide or amino acids capable of constituting the present partial peptide with the remainder, followed by elimination of protecting groups if any from the product. As the publicly known condensation method and the elimination of the protecting groups, mention is made of e.g. the methods described in (1) to (5) below:
(1) M. Bodanszky and M. A. Ondetti, Peptide Synthesis, Interscience Publisher, New York (1966);
(2) Schroeder and Luebke, The Peptide, Academic Press, New York (1965);
(3) Nobuo Izumiya et al., Basis and Experiments in Peptide Synthesis (in Japanese), Maruzen Co., Ltd. (1975);
(4) Haruaki Yajima and Shunpei Sakakibara, Biochemical Experimental Course 1, Protein Chemistry IV, 205 (1977); and
(5) Haruaki Yajima (supervisor), Development of Medicines, a second series, vol. 14, Peptide Synthesis, Hirokawashoten.

After the reaction, the present partial peptide can be isolated and purified by a combination of conventional purification techniques such as solvent extraction, distillation, column chromatography, liquid chromatography and recrystallization. If the partial peptide is obtained in a free form by these methods, the product can be converted into a suitable salt by a publicly known method, or if the product is obtained in a salt form, it can be converted into a free form by a publicly known method.

As a polynucleotide encoding the present receptor protein, any polynucleotides may be used as long as they comprise a base sequence (DNA or RNA, preferably DNA) encoding the aforementioned present receptor protein. As the polynucleotide, there are DNA and a RNA such as a mRNA encoding the present receptor protein, whether double-stranded or single-stranded. In the case of the double-strand, a double-stranded DNA, a double-stranded RNA or a DNA:RNA hybrid may be used. In the case of single-strand, a sense strand (that is, coding strand), or an antisense strand (that is, non-coding strand) may be used.

The mRNA for the present receptor protein can be quantified using a polynucleotide encoding the present receptor protein by a method described in a publicly known publication, for example, Experimental Medicine, Extra Issue, "New PCR and its Applications", 15 (7), 1997, or by its modified method.

As a DNA encoding the present receptor protein, any of a genomic DNA, a genomic DNA library, a cDNA derived form the aforementioned cells or tissues, a cDNA isolated by the publicly known method from a cDNA library derived from the aforementioned cells or tissues, and a synthetic DNA may be used. A vector used for a library may be any of bacteriophage, plasmid, cosmid and phagemide. In addition, amplification can be performed directly by Reverse Transcriptase Polymerase Chain Reaction (abbreviated hereinafter to RT-PCR method) using total RNA or mRNA fraction prepared from the aforementioned cells or tissues.

Specifically, as a DNA encoding the present receptor protein, for example, a DNA comprising a base sequence represented by SEQ ID NO: 2 or SEQ ID NO: 6 or a DNA comprising a base sequence hybridizing under high stringent conditions with a base sequence represented by SEQ ID NO: 2 or SEQ ID NO: 6 and encoding a receptor protein having substantially homogenous physiological activities (for example, ligand binding activity, signal transduction action, etc.) with those of the present receptor protein may be used.

As a DNA which can hybridize with a base sequence represented by SEQ ID NO: 2 or SEQ ID NO: 6, for example, a DNA comprising a base sequence having about 70% or more, preferably about 80% or more, more preferably about 90% or more and most preferably about 95% or more homology with a base sequence represented by SEQ ID NO: 2 or SEQ ID NO: 6, etc. are used.

Hybridization can be carried out according to a publicly known method or its modified method, for example a method described in Molecular Cloning, 2nd ed. (J. Sambrook et al., Cold Spring Harbor Lab. Press, 1989). If a commercial library is used, hybridization can be carried out according to the manufacture's instructions. Preferably, hybridization can be conducted under high stringent conditions.

The high stringent conditions refer to those conditions under which the concentration of sodium is about 19 to 40 mM, preferably about 19 to 20 mM, and the temperature is about 50 to 70 °C, preferably about 60 to 65 °C. In particular, the case where the concentration of sodium is about 19 mM and the temperature is about 65 °C is the most preferable.

More specifically, as a DNA encoding the receptor protein comprising an amino acid sequence represented by SEQ ID NO: 1, a DNA comprising a base sequence represented by SEQ ID NO: 2, etc. are used. Further, as a DNA encoding the receptor protein comprising an amino acid sequence represented by SEQ ID NO: 5, a DNA comprising a base sequence represented by SEQ ID NO: 6, etc. are used.

A polynucleotide comprising a part of a base sequence of a DNA encoding the present receptor protein, or a part of a base sequence complementary with the DNA is meant to include not only a DNA encoding the following present partial peptide but also RNA.

According to the present invention, an antisense polynucleotide (nucleic acid) which can inhibit replication or expression of a G protein coupled receptor protein gene can be designed and synthesized based on base sequence information of a DNA encoding the cloned or determined G protein coupled receptor protein. Such a polynucleotide (nucleic acid) can hybridize with an RNA for a G protein coupled receptor protein gene, can inhibit the synthesis or functions of the RNA, or can regulate or control expression of a G protein coupled receptor protein gene via interaction with a G protein coupled receptor protein-related RNA. A polynucleotide complementary with a selected sequence of a G protein coupled receptor protein-related RNA, and a polynucleotide which can specifically hybridize with a G protein coupled receptor protein-related RNA are useful for regulating and controlling expression of a G protein coupled receptor protein gene *in vivo* and *in vitro*, and useful for treating or diagnosing diseases. A term "correspond" means homologous or complementary with a particular sequence of a nucleotide, a base sequence or nucleic acid including a gene. "Correspond" between a nucleotide, a base sequence or a nucleic acid and a peptide (protein) usually denotes an amino acid of a peptide (protein) under direction induced by a nucleotide (nucleic acid) sequence or its complement. Although 5'-terminal hairpin loop, 5'-terminal 6-base pair repeat, 5'-terminal nontranslation region, polypeptide translation initiating codon, protein coding region, ORF translation initiating codon, 3'-terminal nontranslation region, 3'-terminal palindrome region and 3'-terminal hairpin loop can be selected as a preferable subject region, any region in a G protein coupled receptor gene can be selected as a subject region.

The relationship between an end nucleic acid and a polynucleotide complementary with at least a part of a subject region, and the relationship between a subject and a polynucleotide which can hybridize therewith can be said to be "antisense". As an antisense polynucleotide, there are a polydeoxyribonucleotide comprising 2-deoxy-D-ribose, a polyribonucleotide comprising D-ribose, other type polynucleotide which is a N-glycoside of a purine or pyrimidine base, or other polymer having a non-nucleotide skeleton (for example, a commercially available protein nucleic acid and synthetic sequence specific nucleic acid polymer) or other polymer comprising a special linkage (provided that the polymer contains their nucleotide having arrangement permitting base pairing and base attachment found in a DNA and an RNA). They may be a double-stranded DNA, a single-stranded DNA, a double-stranded RNA, a single-stranded RNA, or a DNA : RNA hybrid, a non-modified polynucleotide (or non-modified oligonucleotide) or may have the added known modification, for example, may have a label known in the art, or have a cap, or may be methylated, or may have substitution of 1 or more natural nucleotides with similar nucleotides, or may have intramolecular nucleotide modification, for example, may have a non-charged linkage (for example, methylphosphonate, phosphotriester, phosphoramidate, and carbanate), may have a charged linkage or a sulfur-containing linkage (for example, phosphorothioate and phosphorodithioate), for example, may have a side chain group of a protein (nuclease, a nuclease inhibitor, toxin, antibody, signal peptide, poly-L-lysine) or a sugar (for example, monosaccharide) , may have an intercalate compound (for example, acridine and psoralen), may contain a chelate compound (for example, metal, radioactive metal, boron, oxidative metal), may contain an alkylating agent, or may have a modified linkage (for example, α-anomer type nucleic acid). Here, "nucleoside", "nucleotide" and "nucleic acid" include not only those comprising a purine and pyrimidine base but also those having modified other heterocyclic type base. Such modified materials may contain methylated purine and pyrimidine, acylated purine and pyrimidine, or other heterocycle. A modified nucleotide and a modified nucleotide may have a modified sugar moiety and, for example, 1 or more hydroxy groups may be substituted with halogen or an aliphatic group, or converted into a functional group such as ether and amine.

The present antisense polynucleotide (nucleic acid) is an RNA, a DNA, or a modified nucleic acid (RNA, DNA) . Examples of the modified nucleic acid include, but are not limited to, a sulfur derivative and a thiophosphate derivative of nucleic acid, and modified nucleic acids such as polynucleosideamide and oligonucleosideamide which are resistant to degradation. The present antisense nucleic acid can be preferably designed under the following strategy. That is, an antisense nucleic acid in a cell is made more stable, the cell permeability of an antisense nucleic acid is enhanced, affinity for a objective sense strand is made greater and, if any, toxicity of an antisense nucleic acid is made smaller.

A variety of such modifications are known in the art. For example, there is a disclosure in J. Kawakami et al., Pharm. Tech. Japan, vol. 8, p. 247, 1992; vol. 8, p. 395, 1992; S. T. Crooke et al. ed., Antisense Research and Applications, CRC Press, 1993.

The present antisense nucleic acid may be altered, may contain a modified sugar, a base or a linkage, may be supplied in the special form such as a liposome and a microsphere, may be applied in gene therapy, may be given in the addition form. Examples of the antisense nucleic acid which may be used in the addition form include a polycation such as polylysine which neutralizes a charge of phosphate skeleton, and hydrophobic material such as a lipid which enhances the interaction with the cell membrane and increases uptake of nucleic acid (for example, phospholipid and cholesterol). Examples of a preferable lipid to be added include cholesterol and its derivative (for example, cholesteryl chloroformate and cholic acid) . These can be attached to a 3'-terminal or a 5'-terminal of nucleic acid and can be attached via a base, a sugar and an intramolecular nucleoside linkage. An example of other group includes a group for capping and preventing degradation by a nuclease such as exonuclease and RNase. An example of such a group for capping includes, but is not limited to, a glycol such as a protecting group for a hydroxyl group known in the art including polyethylene glycol and tetraethylene glycol.

The inhibitory activity of an antisense nucleic acid can be examined by using the present transformant, the present *in vivo* and *in vitro* gene expression system, or an *in vivo* and *in vitro* translation system for a G protein coupled receptor protein. The nucleic acid can be applied to a cell by a variety of publicly known methods.

As a DNA encoding the present partial peptide, any DNAs may be used as long as they contain a base sequence encoding the aforementioned present partial peptides. In addition, the DNA may be a genomic DNA, a genomic DNA library, a cDNA derived from the aforementioned cells or tissues, a cDNA library derived from the aforementioned cells or tissues, or a synthetic DNA. A vector used in a library may be any of bacteriophage, plasmid, cosmid and phagemid. In addition, the DNA may be amplified directly by Reverse Transcriptase Polymerase Chain Reaction (hereinafterabbreviatedasRT-PCR method) using an mRNA fraction prepared from the aforementioned cells and tissues.

Specifically, as a DNA encoding the present partial peptide, for example, (1) a DNA having a partial base sequence of a DNA having a base sequence represented by SEQ ID NO: 2 or SEQ ID NO: 6, or (2) a DNA having a base sequence which hybridizes with a base sequence represented by SEQ ID NO: 2 or SEQ ID NO: 6 under the highly stringent conditions, and having a partial base sequence of a DNA encoding a receptor protein having the substantially homogeneous activities (for example, ligand binding activity and signal transduction action) to those of the present receptor protein peptide are used.

As a DNA which can hybridize with a base sequence represented by SEQ ID NO: 2 or SEQ ID NO: 6, for example, a DNA comprising a base sequence having about 70% or more, preferably about 80% or more, more preferably about 90% or more, most preferably about 95% or more homology with a base sequence represented by SEQ ID NO: 2 or SEQ ID NO: 6 is used.

As a means of cloning the DNA coding completely for the present receptor protein or a partial peptide thereof (hereinafter abbreviated as present receptor protein in some cases), it is possible to use amplification by the PCR method using synthetic DNA primers having a partial base sequence for the present receptor protein as well as selection by hybridization of the DNA integrated in a suitable vector with a labeled DNA fragment or synthetic DNA encoding a part or the whole of the present receptor protein. Hybridization can be carried out according to a method described in, for example, Molecular Cloning, 2nd ed. (J. Sambrook et al., Cold Spring Harbor Lab. Press, 1989). If a commercial library is used, hybridization can be carried out according to the manufacture's instructions.

Base conversion of DNA sequence can be carried out by a publicly known method, for example by the Gupped duplex method or Kunkel method or its modified method by using a known kit such as Mutant™-G (Takara Shuzo Co., Ltd.) or Mutant™-K (Takara Shuzo Co., Ltd.).

A DNA encoding the cloned receptor protein can be used as it is depending on the object, or if desired, by digesting it with a restriction enzyme or adding a linker thereto. The DNA may have ATG as a translation initiation codon at the 5'-terminal thereof and TAA, TGA or TAG as a translation termination codon at the 3'-terminal thereof. These translation initiation and termination codons can also be added to the DNA using a suitable synthetic DNA adaptor.

An expression vector for the present receptor protein can be manufactured for example by (A) excising the desired DNA fragment off from the DNA encoding the present receptor protein and then (B) ligating said DNA fragment to a region downstream from a promoter in a suitable expression vector.

The vector used includes *Escherichia coli*-derived plasmid (e.g., pBR322, pBR325, pUC12, pUC13), *Bacillus subtilis-*derived plasmid (e.g., pUB110, pTP5, pC194), yeast-derived plasmid (e.g., pSH19, pSH15), bacteriophage such as λ-phage, and animal viruses such as retrovirus, vaccinia virus and Baculovirus, as well as pA1-11, pXT1, pRc/CMV, pRc/RSV, pcDNAI/Neo etc.

The promoter used in the present invention may be any suitable promoter compatible with a host used for the gene expression. For example, when animal cells are used as the host, SRα promoter, SV40 promoter, LTR promoter, CMV promoter, HSV-TK promoter, etc are included. Among these, CMV promoter, SRα promoter, etc. are preferably used.

It is preferable to use trp promoter, lac promoter, recApromoter, λP_{L} promoter, lpp promoter etc. for microorganisms of the genus *Escherichia* as the host, SPO1 promoter, SPO2 promoter, penP promoter etc. for microorganisms of the genus *Bacillus* as the host, and PHO5 promoter, PGK promoter, GAP promoter, ADH promoter etc. for yeasts as the host. When insect cells are used as the host, polyhedron promoter, P10 promoter etc. are preferable.

The expression vector may contain an enhancer, a splicing signal, a poly A-added signal, a selective marker, an SV40 replication origin (also referred to hereinafter as SV40 ori) etc. in addition to the elements described above. The selective marker includes, for example, dihydrofolate reductase (also referred to hereinafter as dhfr) gene [methotrexate (MTX) resistance], ampicillin resistance gene (also referred to hereinafter as Amp^{r}) and neomycin resistance gene (G418 resistance, also referred to hereinafter as Neo) . In particular, if the dhfr gene is used as a selective marker for CHO (dhfr⁻) cells, a transformant carrying the desired gene can also be selected in a thymidine-free medium.

A signal sequence compatible with the host is added as necessary to a base sequence for the N- terminal of the present receptor protein. A Pho A signal sequence, Omp A signal sequence etc. can be utilized for microorganisms of the genus Escherichia used as the host; an α-amylase signal sequence, *subtilisin* signal sequence etc. for microorganisms of the genus *Bacillus* as the host; an MFα signal sequence, SUC2 signal sequence etc. for yeasts as the host; and an insulin signal sequence, α-interferon signal sequence, antibody molecule signal sequence etc . for animal cells as the host.

The thus constructed vector containing the DNA encoding the present receptor protein can be used to manufacture transformants.

The microorganisms of the genus *Escherichia,* the microorganisms of the genus *Bacillus,* yeasts, insect cells, insects, animal cells etc. are used as the host.

The microorganisms of the genus *Escherichia* used include, for example, *Escherichia coli* K12 DH1 (Proc. Natl. Acad. Sci. USA, 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

The microorganisms of the genus *Bacillus* used include, for example, *Bacillus subtilis* MI114 (Gene, 24, 255 (1983)), 207-221 (Journal of Biochemistry, 95, 87 (1984)), etc.

The yeasts used include, for example, *Saccharomyces cerevisiae* AH22, AH22R⁻, NA87-11A, DKD-5D, 20B-12, *SchizoSaccharomyces pombe* NCYC1913, NCYC2036, *Pichia pastoris*, etc.

The insect cells used when the virus is AcNPV include, for example, cells (Spodoptera *frugiperda* cells; Sf cells) from an established cell line derived from caterpillars of *Spodoptera frugiperda,* MG1 cells derived from the midgut in *Trichoplusia ni*, High Five™ cells derived from eggs of *Trichoplusia ni,* cells derived from *Mamestra brassicae* and cells derived from *Estigmena acrea.* When the virus is BmNPV, cells (*Bombyx mori* N cells; BmN cells) from an established cell line derived from silkworms, etc., are used. The Sf cells used include, for example, Sf9 cells (ATCC CRL1711), Sf21 cells (Vaughn, J. L. et al., *In vivo*, 13, 213-217 (1977)), etc.

The insects used include, for example, silkworm larvae (Maeda et al., Nature, 315, 592 (1985)).

The animals cells used include, for example, simian cell COS-7, Vero, Chinese hamster cell CHO (hereinafter abbreviated as CHO cell), dhfr gene-defect Chinese hamster cell CHO (hereinafter abbreviated as CHO (dhfr⁻) cell), mouse L cells, mouse AtT-20, mouse myeloma cells, rat GH3, human FL cells etc.

The microorganisms of the genus *Escherichia* can be transformed according to a method described in e.g. Proc. Natl. Acad. Sci. USA, 69, 2110 (1972) or Gene, 17, 107 (1982).

The microorganisms of the genus *Bacillus* can be transformed according to a method described in e.g. Molecular & General Genetics, 168, 111 (1979), etc.

The yeasts can be transformed according to a method described in e.g. Methods in Enzymology, 194, 182-187 (1991), Proc. Natl. Acad. Sci. USA, 75, 1929 (1978), etc.

The insect cells or insects can be transformed according to a method described in e.g. Bio/Technology, 6, 47-55 (1988), etc.

The animal cells can be transformed according to a method described in e.g. "Saibo Kogaku Bessatsu 8, Shin-Saibo Kogaku Jikken Protocol (Cell Technology, Extra Number 8, New Experimental Protocolin Cell Technology)", 263-267 (1995) (published by Shujunsha), Virology, 52, 456 (1973), etc.

In this manner, a transformant transformed with an expression vector comprising a DNA encoding the G protein coupled receptor protein can be obtained.

When transformants derived from the microorganisms of the genus *Escherichia* or *Bacillus* as the host are cultured, the medium used for their culture is preferably a liquid medium containing a carbon source, a nitrogen source, inorganic matter etc. necessary for growth of the transformants. The carbon source includes, for example, glucose, dextrin, soluble starch, sucrose etc.; the nitrogen source includes, for example,. inorganic or organic materials such as ammonium salts, nitrates, corn steep liquor, peptone, casein, meat extract, soybean cake and potato extract; and the inorganic matter includes, for example, calcium chloride, sodium dihydrogen phosphate, magnesium chloride etc. In addition, yeast extracts, vitamins, growth stimulating factors etc. may be added. The pH value of the medium is desirably about 5 to 8.

For example, the medium for culturing the microorganisms of the genus *Escherichia* is preferably M9 medium containing glucose and casamino acid (Miller, Journal of Experiments in Molecular Genetics, 431-433, Cold Spring Harbor Laboratory, New York, 1972). To permit the promoter to work efficiently, a chemical such as 3β-indolylacrylic acid can be added as necessary. The transformants derived from the microorganisms of the genus *Escherichia* as the host are cultured usually at about 15 to 43 °C for about 3 to 24 hours during which the medium may be aerated or stirred as necessary.

The transformants from the microorganisms of the genus *Bacillus* as the host are cultured usually at about 30 to 40 °C for about 6 to 24 hours during which the medium may be aerated or stirred as necessary.

The medium used for culturing the transformants from yeasts as the host includes, for example, Burkholder minimum medium (Bostian, K. L. et al., Proc. Natl. Acad. Sci. USA, 77, 4505 (1980)) and SD medium containing 0.5% casamino acid (Bitter, G. A. et al., Proc. Natl. Acad. Sci. USA, 81, 5330 (1984)). The pH value of the medium is adjusted preferably to about 5 to 8. The transformants are cultured usually at about 20 to 35 °C for about 24 to 72 hours during which the medium may be aerated or stirred as necessary.

The medium used for culturing the transformants from insect cells or insects as the host includes, for example, a medium prepared by adding inactivated additives such as 10% immobilized bovine serum as necessary to Grace's insect medium (Grace, T. C. C., Nature, 195, 788 (1962)). The pH value of the medium is adjusted preferably to about 6.2 to 6.4. The transformants are cultured usually at about 27 °C for about 3 to 5 days during which the medium may be aerated or stirred as necessary.

The medium used for culturing the transformants from animal cells as the host includes, for example, MEM medium containing about 5 to 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)) etc. The pH value is preferably about 6 to 8. The transformants are cultured usually at about 30 to 40 °C for about 15 to 60 hours during which the medium may be aerated or stirred as necessary.

As described above, the present receptor protein can be produced in the cell membrane of the transformant.

Separation and purification of the present receptor protein from the above culture can be performed for example by the following method.

To extract the present receptor protein from the cultured microorganisms or cells, the cultured microorganisms or cells are collected by a publicly known method, suspended in a suitable buffer, disrupted by sonication, lysozyme and/or freezing and thawing, and centrifuged or filtered to give a crude extract of the receptor protein. The buffer may contain protein denaturants such as urea and guanidine hydrochloride and surfactants such as Triton X-100™. When the receptor protein is secreted into the culture fluid, the supernatant is collected by separating the supernatant from the microorganisms or cells by a publicly known method.

The culture supernatant thus obtained, or the receptor protein contained in the extract, can be purified by a suitable combination of publicly known separation and purification techniques. These publicly known separation and purification techniques make use of a method of utilizing solubility, such as salting-out and solvent precipitation, a method of mainly utilizing a difference in molecular weight, such as dialysis, ultrafiltration, gel filtration, and SDS-polyacrylamide gel electrophoresis, a method of utilizing a difference in electric charge, such as ion-exchange chromatography, a method of utilizing specific affinity, such as affinity chromatography, a method of utilizing a difference in hydrophobicity, such as reverse-phase high performance liquid chromatography, a method of utilizing a difference in isoelectric point, such as isoelectric focusing.

If the receptor protein thus obtained is in a free form, it can be converted into a salt by a publicly known method or its modified method, while if the resulting protein is obtained in the form of a salt, it can be converted into a free protein or another salt by a publicly known method or its modified method.

Before or after purification, the receptor protein produced by the transformants can be arbitrarily modified or its partial polypeptide can be removed by allowing a suitable protein-modifying enzyme to act on the receptor protein. For example, trypsin, chymotrypsin, arginyl endopeptidase, protein kinase or glycosidase is used as the protein-modifying enzyme.

The present receptor protein thus formed or a salt thereof can be measured for its activity by an experiment of binding to a labeled ligand, enzyme immunoassays using specific antibody, etc.

The antibody against the present receptor protein, a partial peptide thereof or a salt thereof may be a polyclonal or monoclonal antibody capable of recognizing the present receptor protein, a partial peptide thereof or a salt thereof.

The antibody against the present receptor protein, a partial peptide thereof or a salt thereof (referred to collectively as the present receptor protein) can be produced by a publicly known method for manufacturing antibody or antiserum by using the present receptor protein as the antigen.

### [Preparation of a monoclonal antibody]

### (a) Preparation of monoclonal antibody-producing cells

The present receptor protein or the like is administered alone or together with a carrier and a diluent into mammals at a site where the antibody can be produced by administration. To enhance the ability of the animals upon administration to produce the antibody, complete Freund's adjuvant or incomplete Freund's adjuvant may be administered. Administration is conducted usually once every 2 to 6 weeks and about 2 to 10 times in total. The mammals used include, for example, monkey, rabbit, dog, guinea pig, mouse, rat, sheep and goat, among which mouse and rat are preferably used.

For preparation of the monoclonal antibody-producing cells, those animals having antibody titer are selected from the warm-blooded animals (e.g. mice) immunized with the antigen, and on the second to fifth day after the final immunization, their spleens or lymph nodes are collected, and the antibody-producing cells contained therein are fused with myeloma cells, whereby monoclonal antibody-producing hybridoma can be prepared. The antibody titer in antiserum can be measured for example by reacting the antiserum with the labeled protein described below and then measuring the activity of the labeling agent bound to the antibody. Fusion can be carried out by a known method such as the method of Keller and Millstein (Nature, 256, 495 (1975)). The fusion stimulator includes polyethylene glycol (PEG) and Sendai virus, and PEG is preferably used.

The myeloma cells include, for example, NS-1, P3U1, SP2/0 etc., among which P3U1 is preferably used. The ratio of the antibody-producing cells (spleen cells) to the myeloma cells used is from about 1 : 1 to about 20 : 1, and cell fusion can be effected efficiently by incubating the cells for about 1 to 10 minutes at 20 to 40 °C, preferably 30 to 37 °C, in the presence of PEG (preferably PEG 1000 to PEG 6000) at a concentration of about 10 to 80%.

The monoclonal antibody-producing hybridoma can be screened by various methods, for example by adding a culture supernatant of the hybridoma to a solid phase (e.g., a microplate) having the antibody to the receptor protein, etc., adsorbed thereon directly or along with a carrier and then adding a radioactive substance- or enzyme-labeled anti-immunoglobulin antibody (which is e.g. an anti-mouse immunoglobulin antibody when mouse cells are subjected to cell fusion) or protein A to detect the monoclonal antibody bound to the solid phase or by adding a culture supernatant of the hybridoma to a solid phase having an anti-immunoglobulin antibody or protein A adsorbed thereon and then adding the receptor protein labeled with a radioactive substance or with an enzyme to detect the monoclonal antibody bound to the solid phase.

The monoclonal antibody can be screened according to a publicly known method or its modified method. Screening can be carried out usually in an animal cell culture medium to which HAT (hypoxanthine, aminopterin, thymidine) was added. The screening and breeding medium may be any medium in which the hybridoma can grow. Examples of such medium include PRMI 1640 medium containing 1 to 20% (preferably 10 to 20%) FBS, GIT medium containing 1 to 10% FBS (Wako Pure Chemical Industries, Ltd.), and a serum-free medium for hybridoma culture (SFM-101, Nissui Pharmaceutical, Co., Ltd.). The culture temperature is usually 20 to 40 °C, preferably about 37 °C. The culture time is usually 5 days to 3 weeks, preferably 1 to 2 weeks. Culture can be conducted usually in 5% CO₂ gas. The antibody titer in a culture supernatant of the hybridoma can be measured in the same manner as in the measurement of the antibody titer in antiserum as described above.

### (b) Purification of the monoclonal antibody

Separation and purification of the monoclonal antibody can be performed according to the conventional method, for example, a method of separating and purifying an immunoglobulin [for example, a salting-out method, an alcohol precipitation method, an isoelectric precipitation method, an electrophoresis method, an absorbing-desorbing method with an ion-exchanger (e.g DEAE), an ultracentrifugation method, a gel filtration method, a specific purifying method for obtaining an antibody by collecting it exclusively on antigen-bound solid phase or on an active adsorbent such as protein A or protein G and then dissociating a bonding thereof to obtain the antibody].

### [Preparation of a polyclonal antibody]

The polyclonal antibody of the present invention can be produced by the publicly known method or its modified method. For example, the polyclonal antibody can be manufactured by making an immune antigen (receptor protein antigen)-carrier protein conjugate, then immunizing a mammal therewith in the same manner as in the method of producing the above monoclonal antibody, collecting a material comprising an antibody to the present receptor protein from the immunized animal, and separating and purifying the antibody.

For the immune antigen-carrier protein conjugate used for immunizing mammals, the type of the carrier protein and the mixing ratio of the carrier to the hapten are not particularly limited insofar as the desired antibody can be efficiently produced by immunization with the antigen crosslinked via the hapten with the carrier, and for example, the conjugate is produced by coupling the hapten with bovine serum albumin, bovine cyloglobulin, keyhole limpet hemocyanin or the like in a ratio of from 1 to about 0.1 to 20, preferably from 1 to about 1 to 5.

The hapten can be coupled with the carrier by use of various condensation agents such as glutaraldehyde, carbodiimide, maleimide-activated ester, and activated ester reagents containing thiol group and dithiopyridyl group.

The resulting condensation product is administered alone or together with a carrier and a diluent into mammals at a site where the antibody can be produced. To enhance the ability of the animals upon administration to produce the antibody, complete Freund's adjuvant or incomplete Freund's adjuvant may be administered. Administration is conducted usually once every 2 to 6 weeks and about 3 to 10 times in total.

The polyclonal antibody can be collected form blood, ascites etc. preferably blood in the mammals immunized by the method described above.

The polyclonal antibody titer in antiserum can be measured in the same manner as in the measurement of the antibody titer in antiserum as described above. Separation and purification of the polyclonal antibody can be carried out by a method of separating and purifying immunoglobulin, which is similar to the separation and purification of the monoclonal antibody as described above.

The present receptor protein, a partial peptide thereof or a salt thereof, and a DNA encoding the same can be used in (1) determination of a ligand (agonist) for the present receptor protein, (2) an agent for preventing and/or treating diseases associated with the dysfunction of the present G protein coupled receptor protein, (3) a genetic diagnostic agent, (4) quantification of a ligand for the present G protein coupled receptor protein, (5) screening for a compound (agonist, antagonist) which alters the binding property of the present G protein coupled receptor protein with its ligand, (6) an agent for preventing and/or treating various diseases, which comprises a compound (agonist, antagonist) which alters the binding property of the present G protein coupled receptor protein with its ligand, (7) quantification of the present receptor protein, a partial peptide thereof, or a salt thereof, (8) neutralization by an antibody against the present receptor protein, a partial peptide thereof or a salt thereof, (9) preparation of a non-human animal carrying a DNA encoding the present G protein coupled receptor protein.

In particular, by using a receptor binding assay system using an expression system of the present recombinant G protein coupled receptor protein, a compound which alters binding property of a ligand with a G protein coupled receptor specific for a human and a mammal (for example, agonist and antagonist) can be screened, and the agonist or the antagonist can be used as an agent for preventing or treating various diseases.

The uses of the present receptor protein or a partial peptide thereof or a salt thereof (hereinafter abbreviated as present receptor protein etc. in some cases), a DNA encoding the present receptor protein or a partial peptide thereof (hereinafter abbreviated as present DNA in some cases) and an antibody to the present receptor protein etc. (hereinafter abbreviated as present antibody in some cases) will be explained specifically below.

### (1) Determination of a ligand (agonist) for the present G protein coupled receptor protein

The present receptor protein or a salt thereof or the present partial peptide or a salt thereof is useful as a reagent for searching or determining a ligand (agonist) for the present receptor protein or a salt thereof.

That is, the present invention provides a method for determining a ligand for the present receptor protein, which comprises contacting the present receptor protein or a salt thereof or the present partial peptide or a salt thereof, with a test compound.

As the test compound, the publicly known ligands (for example, angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α- and β-chemokine (for example, IL-8, GROα, GROβ, GROγ, NAP-2, ENA-78, PF4, IP10, GCP-2, MCP-1, MC14, MCP-3, I-309, MIP1α, MIP-1β, RANTES), endothelin, enterogastrin, histamine, neurotensin, TRH, pancreatic polypeptide, galanin or LPA, as well as a tissue extract and a cell culture supernatant of a human or a mammal (for example, mouse, rat, pig, cow, sheep and monkey) are used. For example, a single ligand can be finally obtained by adding the tissue extract or the cell culture supernatant to the present receptor protein, and fractionating while measuring the cell stimulating activity.

More particularly, a method for determining the present ligand is a method for determining a compound (for example, peptide, protein, non-peptide compound, synthetic compound and fermentation product) or a salt thereof having the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) by binding with the present receptor protein, by using the present receptor protein or a partial peptide thereof or a salt thereof, or by constructing an expression system for a recombinant receptor protein, and using a receptor binding assay system employing the expression system.

The method for determining a ligand according to the present invention comprises measuring an amount of a test compound bound to the present receptor protein or a partial peptide, or the cell stimulating activity when the receptor protein or a partial peptide thereof is contacted with a test compound.

More particularly, the present invention provides:
(1) a method for determining a ligand for the present receptor protein or a salt thereof, which comprises measuring an amount of a labeled test compound bound to the present receptor protein or a salt thereof, or the present partial peptide or a salt thereof in the case where a labeled test compound is contacted with the receptor protein or a salt thereof or the partial peptide or a salt thereof,
(2) a method for determining a ligand for the present receptor protein or a salt thereof, which comprises measuring an amount of a labeled test compound bound to a cell comprising the present receptor protein or a membrane fraction of the cell in the case where a labeled test compound is contacted with the cell or the membrane fraction,
(3) a method for determining a ligand for the present receptor protein, which comprises measuring an amount of a labeled test compound bound to the receptor protein or a salt thereof in the case where a labeled test compound is contacted with the receptor protein expressed on a cell membrane by culturing a transformant comprising a DNA encoding the present receptor protein,
(4) a method for determining a ligand for the present receptor protein or a salt thereof, which comprises measuring the cell stimulating activity (for example, the activity promoting or inhibiting arachidonic acid release, acetylcholine release, intracellular Ca²⁺ release, intracellular CAMP production, intracellular cGMP production, inositol phosphate production, cell membrane potential fluctuation, intracellular protein phosphorylation, c-fos activation, and reduction of pH) via a receptor protein in the case where a test compound is contacted with a cell comprising the present receptor protein, and
(5) a method for determining a ligand for the present receptor protein or a salt thereof, which comprises measuring the cell stimulating activity (for example, the activity promoting or inhibiting arachidonic acid release, acetylcholine release, intracellular Ca²⁺ release, intracellular CAMP production, intracellular cGMP production, inositol phosphate production, cell membrane potential fluctuation, intracellular protein phosphorylation, c-fos activation, and reduction of pH) via a receptor protein in the case where a test compound is contacted with a receptor protein expressed on a cell membrane by culturing a transformant comprising a DNA encoding the present receptor protein.

In particular, it is preferable that the above experiments (1) to (3) are first carried out to confirm binding of a test compound to the present receptor protein, and then the above experiments (4) to (5) are carried out.

First, as a receptor protein used for the ligand determining method, any receptor proteins may be used as long as they contain the aforementioned present receptor protein or present partial peptide, and a receptor protein which was expressed in a large amount using an animal cell is suitable.

In order to manufacture the present receptor protein, the aforementioned method is used, but it is preferable that preparation is performed by expressing a DNA encoding the receptor protein in a mammalian cell or an insect cell. As a DNA fragment encoding a protein portion of interest, a cDNA is usually used but is not necessarily limited to it. For example, a gene fragment or a synthetic DNA may be used. In order to introduce a DNA fragment encoding the present receptor protein into a host animal cell and express it efficiently, it is preferable that the DNA fragment is integrated in downstream of a polyhedron promoter from nuclear polyhedrosis virus (NPV) belonging to Baculovirus whose host is an insect, SV40-derived promoter, promoter of retrovirus, metallothionein promoter, human heat shock promoter, cytomegalovirus promoter and SRα promoter. Investigation of an amount and quality of the expressed receptor can be conducted by the publicly known method. The investigation can be conducted by, for example, a method described in a reference [Nambi, P. et al., J. Biol. Chem., vol. 267, pp. 19555-19559, 1992].

Therefore, in the present ligand determining method, a material comprising the present receptor protein or a partial peptide thereof or a salt thereof may be a receptor protein or a partial peptide thereof or a salt thereof purified according to the publicly known method, or a cell comprising the receptor protein or a cell membrane fraction thereof may be used.

In the present ligand determining method, when a cell comprising the present receptor protein is used, the cell may be fixed with glutaraldehyde or formalin. The fixing method can be conducted according to the publicly known method.

A cell comprising the present receptor protein refers to a host cell which expressed the present receptor protein and, as the host cell, *Escherichia coli, Bacillus subtilis,* yeast, insect cell and animal cell are used.

A cell membrane fraction refers to a fraction in which a cell membrane obtained by the publicly known method after disruption of a cell is contained in a large amount. As a method for disrupting a cell, there are a method of squeezing a cell with a Potter-Elvehjem type homogenizer, disruption with a Waring blender or Polytron (manufactured by Kinematica), disruption by sonication, and disruption by jetting a cell through a thin nozzle while pressurizing with a French press. For fractionating a cell membrane, a fractionating method by centrifugation force such as a fractionating centrifugation method and density gradient centrifugation method is mainly used. For example, a disrupted cell suspension is centrifuged at a low speed (500 rpm-3000 rpm) in a short time (usually about 1 minute to 10 minutes), the supernatant is centrifuged at a higher speed (15000 rpm-30000 rpm) usually for 30 minutes to 2 hours, and the resulting precipitate is used as a membrane fraction. An expressed receptor protein and membrane components such as cell-derived phospholipid and membrane protein are contained in a large amount in the membrane fraction.

An amount of a receptor protein in a cell comprising the receptor protein or its membrane fraction is preferably 10³-10⁸ molecules, suitably 10⁵-10⁷ molecules per cell. In addition, as an expressed amount is larger, ligand binding activity per membrane fraction (specific activity) is raised, and not only a high sensitive screening system can be constructed but also a large amount of samples can be measured at the same lot.

In order to conduct the above methods (1) to (3) for determining a ligand for the present receptor protein or a salt thereof, a suitable receptor protein fraction and a labeled test compound are used.

As a receptor protein fraction, a natural receptor protein fraction or a recombinant receptor fraction having the equal activity thereto are desirable. Here, equal activity denotes equal ligand binding activity and signal transduction action.

As a labeled test sample, those compounds labeled with [³H], [¹²⁵I], [¹⁴C], [³⁵S] etc., such as angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α- and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES), endothelin, enterogastrin, histamine, neurotensin, TRH, pancreatic polypeptide, galanin, LPA are preferable.

Specifically, in order to conduct a method for determining a ligand for the present receptor protein or a salt thereof, first, a receptor protein preparation is prepared by suspending a cell comprising the present receptor protein or a membrane fraction of the cell in a buffer suitable for the determination method. Any buffers which do not inhibit binding of a ligand with a receptor protein may be used such as phosphate buffer and Tris-HCl buffer, pH 4-10 (desirably pH 6-8). In addition, in order to reduce non-specific binding, surfactants such as CHAPS, Tween-80™ (Kao-Atlas Company), digitonin, and deoxycholate and various proteins such as bovine serum albumin and gelatin may be added to a buffer. Further, in order to suppress degradation of a receptor and a ligand by a protease, a protease inhibitor such as PMSF, leupeptin, E-64 (manufactured by Peptide Laboratory) and pepstain may be added. A test compound labeled with a given amount of (5000 cpm-500000 cpm) of [³H], [¹²⁵I], [¹⁴C] or [³⁵S] is present in 0.01 ml to 10 ml of the receptor solution. In order to know an amount of non-specific binding (NSB), a reaction tube to which an unlabeled test compound has been added in large excess is also prepared. The reaction is conducted at about 0 °C to 50 °C, preferably about 4 °C to 37 °C, for about 20 minutes to 24 hours, desirably about 30 minutes to3hours. After the reaction, the reaction solution is filtered with a glass fiber filter paper and washed with a suitable amount of the same buffer, and the radioactivity remaining in the glass fiber filter paper is measured by a liquid scintillation counter or a γ-counter. A test compound having a count (B - NSB) greater than 0 cpm, as determined by subtracting a non-specific binding amount (NSB) from a total binding amount (B), can be selected as a ligand (agonist) for the present receptor protein or a salt thereof.

In order to conduct the above methods (4) to (5) for determining a ligand for the present receptor protein or a salt thereof, the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via the receptor protein can be measured by the known method or using a commercially available measuring kit. More particularly, first a cell comprising a receptor protein is cultured on a multi-well plate. Upon implementation of ligand determination, a medium and a buffer are exchanged with a fresh medium or a suitable buffer showing no toxicity to a cell in advance, a test compound is added and incubated for a given time, a cell is extracted or the supernatant is recovered, and the produced product is quantified according to respective methods. When the production of a substance as an index for the cell stimulating activity (for example, arachidonic acid) is difficult to be assayed due to a degrading enzyme contained in a cell, an assay may be conducted by adding an inhibitor for the degrading enzyme. In addition, the activity such as inhibition of cAMP production can be detected as production inhibiting action in a cell whose basal production amount has been increased by froscolin.

A kit for determining a ligand which binds to the present receptor protein or a salt thereof comprises the present receptor protein or a salt thereof, the present partial peptide or a salt thereof, a cell comprising the present receptor protein, or a membrane fraction of a cell comprising the present receptor protein.

Examples of the present ligand determining kit are as follows.

### 1. Ligand determining reagent

(a) Buffers for measuring and washing
   Hanks' Balanced Salt Solution (manufactured by Gibco) supplemented with 0.05% bovine serum albumin (manufactured by Sigma)
   Buffers may be sterilized with a filter having a pore diameter of 0.45 µm and stored at 4 °C, or may be prepared at use.
(b) G protein coupled receptor protein preparation
   CHO cells which have expressed the present receptor protein are subcultured in a 12-well plate at a density of 5×10⁵ cells/well, and then cultured at 37 °C in 5% CO₂ and 95% air for 2 days.
(c) Labeled test compound
   A compound labeled with commercially available [³H], [¹²⁵I], [¹⁴C] or [³⁵S], or labeled with a suitable method
   Its aqueous solution is stored at 4 °C or -20 °C, and diluted with the measuring buffer to a concentration of 1 µM just at use. A test compound which is sparingly soluble in water is dissolved in dimethyl formamide, DMSO or methanol.
(d) Unlabeled test compound
   The same unlabeled compound as the labeled compound is prepared at the 100- to 1000-fold concentration.

### 2. Measuring method

(a) CHO cells expressing the present receptor protein cultured in a 12-well tissue culturing plate are washed twice with 1 ml of the measuring buffer, and 490 µl of measuring buffer is added to each well.
(b) 5 µl of a labeled test compound is added and reacted at room temperature for 1 hour. In order to know an amount of non-specific binding, 5 µl of a non-labeled test compound is added.
(c) The reaction solution is removed, and the plate is washed three times with 1 ml of the washing buffer. A labeled test compound bound to a cell is dissolved in 0.2 N NaOH-1% SDS, and mixed with 4 ml of a liquid scintillator A (manufactured by Wako Pure Chemical Industries, Ltd.).
(d) The radioactivity is measured using a liquid scintillation counter (manufactured by Beckmann).

As a ligand which can bind to the present receptor protein or a salt thereof, for example, there are substances present specifically in brain, pituitary gland and pancreas, and more particularly, angiotensin, bombesin, canabinoide, cholecystokinin, glutamine, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amyline, bradykinin, CGRP (calcitonin gene related peptide), leukotriene, pancreastatin, prostaglandin, thromboxane, adenosine, adrenaline, α- and β-chemokine (for example, IL-8, GROα, GROβ, GROγ, NAP-2, ENA-78, PF4, IP10, GCP-2, MCP-1, HC14, MCP-3, 1-309, MIP1α, MIP-1β, RANTES), endothelin, enterogastrin, histamine, neurotensin, TRH, pancreatic polypeptide, galanin and LPA are used. More specifically, LPA (lysophosphatidic acid) is used. (2) An agent for preventing and/or treating diseases associated with the dysfunction of the present G protein coupled receptor protein.

In the above (1) method, when a ligand for the present receptor protein is revealed, (a) the present receptor protein or (b) a DNA encoding the receptor protein can be used as a pharmaceutical composition for preventing and/or treating diseases associated with the dysfunction of the present receptor protein, depending upon the action harbored by the ligand.

For example, when there is a patient for whom the physiological action of the ligand cannot be expected (lack of the receptor protein) due to a decrease in the present receptor protein in vivo, an amount of a receptor protein in vivo of the patient can be increased and the action of a ligand can be sufficiently exerted by (i) compensating for an amount of the receptor protein by administering the present receptor protein to the patient or by (ii) (a) administering a DNA encoding the present receptor protein to the patient to express the DNA, or (b) inserting a DNA encoding the present receptor protein into a subject cell to express the DNA, and transplanting the cell into the patient. That is, a DNA encoding the present receptor protein is useful as a safe and low toxic drug for preventing and/or treating diseases associated with the dysfunction of the present receptor protein.

The present receptor protein is recognized to have about 50% homology at the amino acid sequence level, with EDG-2 receptor that is a G protein coupled receptor protein.

The present receptor protein is useful for preventing and/or treating center diseases (for example, Alzheimer's disease, dementia, ingestion disturbance etc.), inflammatory diseases (for example, allergy, asthma, rheumatism etc.), diseases in circulatory organs (for example, hypertension, cardiac hypertrophy, angina pectoris, arteriosclerosis etc.), cancers (for example, non-small cell lung cancer, ovary cancer, prostate cancer, stomach cancer, bladder cancer, breast cancer, cervical cancer, colon cancer, rectal cancer etc.), diabetes etc.

When the present receptor protein is used as the preventing and treating agent, it can be formulated according to the conventional means.

On the other hand, when a DNA encoding the present receptor protein (hereinafter abbreviated as present DNA in some cases) is used as the above preventing and treating agent, the present DNA can be implemented according to the conventional means, alone or after inserted into a suitable vector such as a retrovirus vector, an adenovirus vector and an adenovirus-associated virus vector. The present DNA can be administered alone or together with an auxiliary agent for promoting uptake by a catheter such as a gene gun or a hydrogel.

For example, (a) the present receptor protein or (b) a DNA encoding the receptor protein can be used orally as tablets coated with a sugar-coating if necessary, capsules, elixirs or microcapsules, or can be used parenterally in the form of an injection such as a sterile solution or a suspension with water or other pharmaceutically acceptable solution. For example, (a) the present receptor protein or (b) a DNA encoding the receptor protein can be formed by blending with physiologically acceptable carriers, flavors, excipients, vehicles, preservatives, stabilizers and binders, into a unit dosage form required for the generally recognized preparation implementation. An amount of an effective ingredient in these pharmaceutical preparations is such that a suitable volume in an indicated range can be obtained.

The additives miscible with the tablets, capsules etc. include, for example, binders such as gelatin, corn starch, tragacanth and gum arabic, excipients such as crystalline cellulose, swelling agents such as corn starch, gelatin and arginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water. The aqueous solution for injection includes physiological saline or an isotonic solution containing glucose and other supplementary agents (e.g., D-sorbitol, D-mannitol, sodium chloride etc.), and may be used in combination with suitable solubilizers such as alcohols (e.g., ethanol etc.), polyalcohols (e.g., propylene glycol, polyethylene glycol etc.) and nonionic surfactants (e.g., Polysorbate 80™, HCO-50 etc.). The oily solution includes sesame oil, soybean oil etc., and may be used in combination with solubilizer such as benzyl benzoate, benzyl alcohol etc.

Further, the above preventing or treating agent may be blended, for example, with the buffer (e.g., phosphate buffer, sodium acetate buffer etc.), soothing agents (e.g., benzalkonium chloride, procaine hydrochloride etc.), stabilizers (e.g., human serum albumin, polyethylene glycol etc.), preservatives (e.g., benzyl alcohol, phenol etc.), antioxidants etc. Usually, the prepared injection is introduced into suitable ampoules.

Since the thus obtained preparation is safe and low toxic, it can be administered, for example, to a human or a mammal (e.g., rat, mouse, rabbit, sheep, pig, cow, cat, dog, monkey, etc.).

A dose of the present receptor protein is different depending on an administration subject, a subject organ, symptom and an administration method, and in the case of oral administration, the dose is usually about 0.1 to 100 mg, preferably about 1.0 to 50 mg and more preferably about 1.0 to 20 mg per day in a patient (60 kg) with arteriosclerosis. When parenterally administered, one time dose is different depending upon an administration subject, a subject organ, symptom and an administration method and, for example, in the form of an injection, it is advantageous to administer about 0.01 to 30 mg, preferably about 0.1 to 20 mg and more preferably about 0.1 to 10 mg per day through intravenous injection in a patient (60 kg) with arteriosclerosis. In the case of other animals, an amount calculated per 60 kg can be administered.

A dose of the present DNA is different depending on an administration subject, a subject organ, symptom and an administration method, and in the case of oral administration, the dose is usually about 0.1 to 100 mg, preferably about 1.0 to 50 mg and more preferably about 1.0 to 20 mg per day in a patient (60 kg) with arteriosclerosis. When parenterally administered, one time dose is different depending upon an administration subject, a subject organ, symptom and an administration method and, for example, in the form of an injection, it is advantageous to administer about 0.01 to 30 mg, preferably about 0.1 to 20 mg and more preferably about 0.1 to 10 mg per day through intravenous injection in a patient (60 kg) with arteriosclerosis. In the case of other animals, an amount calculated per 60 kg can be administered.

### (3) Genetic diagnostic agent

Since the present DNA can detect abnormality of a DNA or an mRNA encoding the present receptor protein or a partial peptide thereof (gene abnormality) in a human or a mammal (e.g., rat, mouse, rabbit, sheep, pig, cow, cat, dog, monkey etc.) by using it as a probe, it is useful as a gene diagnostic agent for damage, mutation or expression reduction of the DNA or mRNA, or increase or excess expression of the DNA or mRNA.

The above gene diagnosis using the present DNA can be carried out, for example, by the publicly known method of Northern hybridization or PCR-SSCP (Genomics, vol. 5, pp. 874-879 (1989), Proceedings of the National Academy of Sciences of the United States of America, vol. 86, pp. 2766-2770 (1989)).

### (4) A method for quantifying a ligand for the present G protein coupled receptor protein

Since the present receptor protein and the like have the property of binding to a ligand, the ligand concentration in vivo can be quantified with a better sensitivity.

The present quantification method can be used by combining, for example, with a competition method. That is, the ligand concentration in a test compound can be measured by contacting a test compound with the present receptor protein and the like. More particularly, for example, the method can be used according to a method described in the following (a) or (b) or its modified method.
(a) "Radioimmunoassay" ed. by Hiroshi Irie (Kodansha, published in 1974)
(b) "Radioimmunoassay", "second series" ed. by Hiroshi Irie (Kodansha, published in 1979)

### (5) A method for screening a compound (such as agonist and antagonist) which alters binding property of the present G protein coupled receptor protein with a ligand

A compound (for example, peptide, protein, non-peptide compound, synthetic compound, and fermentation product) or a salt thereof can be efficiently screened by using the present receptor protein and the like, or constructing an expression system for a recombinant receptor protein and using a receptor binding assay system using the expression system.

Such a compound includes (a) a compound having the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a G protein coupled receptor (so called agonist for the present receptor protein), (b) a compound having no cell stimulating activity (so called antagonist for the present receptorprotein), (c) a compound which enhances binding of a ligand with the present G protein coupled receptor protein, or (d) a compound which reduces binding of a ligand with the present G protein coupled receptor protein (the above (a) compound is screened preferably by the aforementioned ligand determining method).

That is, the present invention provides a method for screening a compound or a salt thereof which alters binding property of a ligand with the present receptor protein or a partial peptide thereof or a salt thereof, which comprises comparing (i) the case where the present receptor protein or a partial peptide thereof or a salt thereof is contacted with a ligand, with (ii) the case where the present receptor protein or a partial peptide thereof or a salt thereof is contacted with a ligand and a test compound.

The present screening method is characterized in that, for example, an amount of a ligand bound to the receptor protein and the like, and the cell stimulating activity in the case of (i) and (ii) are measured and compared.

More particularly, the present invention provides:
(a) a method for screening a compound or a salt thereof which alters binding property of a ligand with the present receptor protein and the like, which comprises measuring and comparing an amount of a labeled ligand bound to the receptor protein and the like in the case where a labeled ligand is contacted with the present receptor protein and the like and the case where a labeled ligand and a test compound are contacted with the present receptor protein and the like,
(b) a method for screening a compound or a salt thereof which alters binding property of a ligand with the present receptor protein and the like, which comprises measuring and comparing an amount of a labeled ligand bound to a cell comprising the present receptor protein and the like or a membrane fraction of the cell in the case where a labeled ligand is contacted with the cell or the membrane fraction and the case where a labeled ligand and a test compound are contacted with the cell or the membrane fraction,
(c) a method for screening a compound or a salt thereof which alters binding property of a ligand with the present receptor protein and the like, which comprises measuring and comparing an amount of a labeled ligand bound to a receptor protein and the like expressed on a cell membrane by culturing a transformant comprising the present DNA in the case where a labeled ligand is contacted with the receptor protein and the like and the case where a labeled ligand and a test compound are contacted with the receptor protein and the like,
(d) a method for screening a compound or a salt thereof which alters binding property of a ligand with the present receptor protein and the like, which comprises measuring and comparing the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a receptor in the case where a compound which activates the present receptor protein and the like (for example, a ligand for the present receptor protein and the like) is contacted with a cell comprising the present receptor protein and the like and the case where a compound which activates the present receptor protein and the like and a test compound are contacted with a cell comprising the present receptor protein and the like, and
(e) a method for screening a compound or a salt thereof which alters binding property of a ligand with the present receptor protein and the like, which comprises measuring and comparing the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a receptor in the case where a compound which activates the present receptor protein and the like (for example, a ligand for the present receptor protein and the like) is contacted with the present receptor protein and the like expressed on a cell membrane by culturing a transformant comprising the present DNA and the cases where a compound which activates the present receptor protein and the like and a test compound are contacted with the present receptor protein and the like expressed on a cell membrane by culturing a transformant comprising the present DNA.

For screening a G protein coupled receptor agonist or antagonist before the present receptor protein and the like were obtained, a method was used in which a cell, a tissue or a cell membrane fraction comprising a Gprotein coupled receptor protein is used to confirm whether a screened compound actually inhibits binding of a G protein coupled receptor protein with a ligand. However, when a cell, a tissue or a cell membrane fraction is used as it is, other receptor proteins are present in admixture therewith, thus making it difficult to actually screen an agonist or an antagonist for the receptor protein of interest.

However, for example by using the present receptor protein, a compound which inhibits binding of a ligand with a G protein coupled receptor protein can be efficiently screened. Further, whether the screened compound is an agonist or an antagonist can be simply assessed.

Hereinafter, the screening method of the present invention is specifically described.

First, as the present receptor protein used in the screening method of the present invention, any proteins may be used as long as they contain the aforementioned present receptor protein and the like, but extracts fromorgans in mammals carrying the present receptor protein and the like are preferable. To obtain a large amount of the receptor protein for use in screening, however, the receptor protein and the like expressed in a large amount by a recombinant is suitable.

In order to manufacture the present receptor protein, the aforementioned method is used, but it is preferable that the present DNA is expressed in a mammalian cell or an insect cell. As a DNA fragment encoding a protein portion of interest, a cDNA is usually used but is not necessarily limited to it. For example, a gene fragment or a synthetic DNA may be used. In order to introduce a DNA fragment encoding the present receptor protein into a host animal cell and express it efficiently, it is preferable that the DNA fragment is integrated in a region downstream from a polyhedron promoter from nuclear polyhedrosis virus (NPV) belonging to Baculovirus whose host is an insect, SV40-derived promoter, promoter of retrovirus, metallothionein promoter, human heat shock promoter, cytomegalovirus promoter and SRα promoter. Investigation of an amount and quality of the expressed receptor can be conducted by the publicly known method. The investigation can be conducted by, for example, a method described in a reference [Nambi, P. et al., J. Biol. Chem., vol. 267, pp. 19555-19559, 1992].

Therefore, in the screening method of the present invention, a material comprising the present receptor protein and the like may be the receptor protein and the like which were purified according to the publicly known method, or a cell comprising said receptor protein may be used, or a membrane fraction of a cell comprising said receptor protein may be used.

In the screening method of the present invention, when a cell comprising the present receptor protein is used, the cell maybe fixed with glutaraldehyde or formalin. The fixing method can be conducted according to the publicly known method.

A cell comprising the present receptor protein refers to a host cell which has expressed the present receptor protein and, as the host cell, *Escherichia coli, Bacillus subtilis,* yeast, insect cell and animal cell are preferable.

A cell membrane fraction refers to a fraction in which a cell membrane obtained by the publicly known method after disruption of a cell is contained in a large amount. As a method for disrupting a cell, there are a method of squeezing a cell with a Potter-Elvehjemtypehomogenizer, disruption with a Waring blender or Polytron (manufactured by Kinematica), disruption by sonication, and disruption by jetting a cell through a thin nozzle while pressurizing with a French press. For fractionating a cell membrane, a fractionating method by centrifugation force such as a fractionating centrifugation method and density gradient centrifugation method is mainly used. For example, a disrupted cell suspension is centrifuged at a low speed (500 rpm-3000 rpm) in a short time (usually about 1 minute to 10 minutes), the supernatant is centrifuged at a higher speed (15000 rpm-30000 rpm) usually for 30 minutes to 2 hours, and the resulting precipitate is used as a membrane fraction. An expressed present receptor protein and membrane components such as cell-derived phospholipid and membrane protein are contained in a large amount in the membrane fraction.

An amount of the receptor protein in a cell comprising the receptor protein or its membrane fraction is preferably 10³-10⁸ molecules, suitably 10⁵-10⁷ molecules per cell. In addition, as an expressed amount is larger, the ligand binding activity per membrane fraction (specific activity) is raised, thus enabling not only construction of a high sensitive screening system but also measurement of a large number of samples in the same lot.

In order to conduct the above methods (a) to (c) for screening a compound which alters binding property of a ligand with the present receptor protein, for example, a suitable receptor protein fraction and a labeled ligand are necessary.

As the receptor protein fraction, a natural receptor protein fraction or a recombinant receptor protein fraction having the equal activity thereto are desirable. Here, equal activity denotes an equal ligand-binding activity, signal transduction action, etc.

As the labeled ligand, a labeled ligand, a labeled ligand modified compound, etc. are used. For example, the ligand labeled with [³H], [¹²⁵I], [¹⁴C], [³⁵S] or the like can be used.

Specifically, in order to conduct screening of a compound which alters binding property of a ligand with the present receptor protein, first, the receptor protein preparation is prepared by suspending a cell comprising the present receptor protein or a membrane fraction of the cell in a buffer suitable for screening. Any buffers which do not inhibit binding of a ligand with the present receptor protein, for example phosphate buffer and Tris-HCl buffer, pH4-10 (desirably pH 6-8), may be used. In addition, in order to decrease non-specific binding, surfactants such as CHAPS, Tween-80™ (Kao-Atlas Company) , digitonin, and deoxycholate may be added to the buffer. Further, in order to suppress degradation of the receptor and ligand by a protease, a protease inhibitor such as PMSF, leupeptin, E-64 (manufactured by Peptide Laboratory) and pepstain may be added. The ligand labeled with a given amount (5000 cpm-500000 cpm) of a radioactive substance is added to 0.01 ml to 10 ml of the receptor solution, and simultaneously 10⁻⁴ to 10⁻¹⁰ M test compound is allowed to be coexist. In order to know an amount of non-specific binding (NSB), a reaction tube to which the unlabeled ligand has been added in large excess is also prepared. The reaction is conducted at about 0 °C to 50 °C, preferably about 4 °C to 37 °C, for about 20 minutes to 24 hours, desirably about 30 minutes to 3 hours. After the reaction, the reaction solution is filtered with a glass fiber filter paper and washed with a suitable amount of the same buffer, and the radioactivity remaining in the glass fiber filter paper is measured by a liquid scintillation counter or a γ-counter. Assuming that the count (B₀-NSB) obtained by subtracting the amount of non-specific binding (NSB) from the count (B₀) in the absence of an antagonist is 100%, a test compound by which the amount of specific binding (B-NSB) is reduced, for example, to 50% or less can be selected as a candidate compound having an antagonistic inhibitory ability.

In order to conduct the above methods (d) to (e) for screening a compound which alters binding a ligand with the present receptor protein, the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via the receptor protein can be measured by the publicly known method or using a commercially available measuring kit.

More particularly, first a cell comprising the present receptor protein and the like is cultured on a multi-well plate. For conducting screening, a medium and a buffer are exchanged with a fresh medium or a suitable buffer showing no toxicity to a cell in advance, a test compound is added and incubated for a given period, a cell is extracted or the supernatant is recovered, and the produced product is quantified according to respective methods. When the production of a substance as an index for the cell stimulating activity (for example, arachidonic acid) is difficult to be assayed due to a degrading enzyme contained in a cell, an assay may be conducted by adding an inhibitor for the degrading enzyme. In addition, the activity such as inhibition of cAMP production can be detected as production inhibiting action in a cell whose basal production has been increased by fros*colin*.

In order to conduct screening by measuring the cell stimulating activity, suitable cells expressing the receptor protein are used. As cells expressing the present receptor protein and the like, a cell line comprising the natural present receptor protein etc. and a cell line expressing the aforementioned recombinant receptor protein etc. are desirable.

As the test compound, for example, peptides, proteins, non-peptide compounds, synthetic compounds, fermentation products, cellular extracts, plant extracts, animal tissue extracts, etc. are used, and these compounds may be novel compounds or known compounds.

A kit for screening a compound or a salt thereof which alters binding property of a ligand with the present receptor protein and the like comprises the present receptor protein and the like, a cell comprising the present receptor protein and the like, or a membrane fraction from cells containing the present receptor protein and the like.

Examples of the present screening kit are as follows.
1. Reagents for screening
   (a) Buffers for measuring and washing
      Hanks' Balanced Salt Solution (manufactured by Gibco) supplemented with 0.05% bovine serum albumin (manufactured by Sigma)
      Buffers may be sterile-filtered with a filter having a pore diameter of 0.45 µm and stored at 4°C, or may be prepared just at use.
   (b) G protein coupled receptor protein preparation
      CHO cells which have expressed the present receptor protein are subcultured in a 12-well plate at a density of 5×10⁵ cells/well, and cultured at 37 °C in 5% CO₂ and 95% air for 2 days.
   (c) Labeled ligand
      An aqueous solution of a ligand labeled with commercially available [³H], [¹²⁵I], [¹⁴C] or [³⁵S], is stored at 4 °C or -20 °C, and diluted with a measuring buffer to 1 µM just at use.
   (d) Ligand standard solution
      The ligand is dissolved at a concentration of 1 mM in PBS containing 0.1% bovine serum albumin (manufactured by Sigma) and stored at -20 °C.
2. Measuring method
   (a) CHO cells expressing the present receptor protein cultured in a 12-well tissue culturing plate are washed twice with 1 ml of a measuring buffer, and 490 µl measuring buffer is added to each well.
   (b) After 5 µl of 10⁻³ to 10⁻¹⁰ M test compound solution is added thereto, 5 µl labeled ligand is added thereto and reacted at room temperature for 1 hour. In order to know an amount of non-specific binding, 5 µl of 10⁻³ M ligand is added in place of the test compound.
   (c) The reaction solution is removed, and the plate is washed three times with 1 ml of a washing buffer. A labeled ligand bound to a cell is dissolved in 0.2 N NaOH-1% SDS, and mixed with 4 ml of a liquid scintillator A (manufactured by Wako Pure Chemical Industries, Ltd.).
   (d) The radioactivity is measured using a liquid scintillation counter (manufactured by Beckmann), to determine percent maximum binding (PMB) in the following equation:
      PMB = [(B-NSB)/(B₀-NSB)] x 100
      PMB: Percent maximum binding
      B: Value when a sample was added
      NSB: Non-specific binding
      B₀: Maximum binding

The compound or a salt thereof obtained by using the screening method or the kit for screening according to the present invention is a compound which alters binding property of a ligand with the present receptor protein and the like, and specifically it is (a) a compound having the cell stimulating activity (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH) via a G protein coupled receptor (so called agonist for the present receptor protein), (b) a compound having none of the cell stimulating activity (so called antagonist for the present receptor protein), (c) a compound which enhances binding of a ligand with the present G protein coupled receptor protein, or (d) a compound which reduces binding of a ligand with the present G protein coupled receptor protein.

As the compound, for example, peptides, proteins, non-peptide compounds, synthetic compounds, fermentation products, etc. are exemplified, and these compounds may be novel compounds or known compounds.

The agonist for the present receptor protein and the like has the same action as the physiological activity possessed by a ligand for the present receptor protein and the like, and is thus useful as a safe and low toxic drug, depending on the activity of said ligand.

The antagonist for the present receptor protein and the like can suppress the physiological activity possessed by a ligand for the present receptor protein and the like and is thus useful as a safe and low toxic drug for suppressing the activity of said ligand.

A compound which enhances binding of a ligand with the present G protein coupled receptor protein is useful as a safe and low toxic drug for enhancing the physiological activity possessed by a ligand for the present receptor protein and the like.

A compound which reduces binding of a ligand with the present G protein coupled receptor protein is useful as a safe and low toxic pharmaceutical composition for reducing the physiological activity possessed by a ligand for the present receptor protein and the like.

When the compound or a salt thereof obtained by using the screening method or the kit for screening according to the present invention is used as the aforementioned pharmaceutical composition, it can be formed into a pharmaceutical preparation by the conventional means. For example, the compound may be formulated into tablets, capsules, elixirs, microcapsules, sterile solution and suspension as in the aforementioned pharmaceutical composition comprising the present DNA.

Since the thus obtained preparations are safe and low toxic, they can be administered, for example, to a human or a mammal (for example, rat, mouse, rabbit, sheep, pig, cow, cat, dog and monkey).

A dose of the compound or its salt is different depending on an administration subject, a subject organ, symptom and an administration method, and in the case of oral administration, the dose is usually about 0.1 to 100 mg, preferably about 1.0 to 50 mg and more preferably about 1.0 to 20 mg per day in a patient (60 kg) with arteriosclerosis. When parenterally administered, one time dose is different depending upon an administration subject, a subject organ, symptom and an administration method and, for example, in the form of an injection, it is advantageous to administer about 0.01 to 30 mg, preferably about 0.1 to 20 mg and more preferably about 0.1 to 10 mg per day through intravenous injection in a patient (60 kg) with arteriosclerosis. In the case of other animals, an amount calculated per 60 kg can be administered. (6) An agent for preventing and/or treating various diseases, comprising a compound (agonist, antagonist) which alters binding property of the present G protein coupled receptor protein with its ligand

The present receptor protein plays an important role invivo such as central functions as described above. Therefore, a compound (agonist, antagonist) which alters binding property of the present receptor protein with its ligand can be used as an agent for preventing and/or treating diseases associated with the dysfunctions of the present receptor protein.

When said compound is used as an agent for preventing and/or treating diseases associated with the dysfunctions of the present receptor protein, it can be formed into a pharmaceutical composition by the conventional means.

For example, said compound can be used orally as tablets coated with a sugar-coating if necessary, capsules, elixirs or microcapsules, or can be used parenterally in the form of an injection such as a sterile solution or a suspension with water or other pharmaceutically acceptable solution. For example, said compound can be manufactured by blending with physiologically acceptable carriers, flavors, excipients, vehicles, preservatives, stabilizers and binders, into a unit dosage form required for the generally recognized preparation implementation. An amount of an effective ingredient in these pharmaceutical preparations is such that a suitable volume in an indicated range can be obtained.

The additives miscible with the tablets, capsules etc. include, for example, binders such as gelatin, corn starch, tragacanth and gum arabic, excipients such as crystalline cellulose, swelling agents such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water. The aqueous solution for injection includes physiological saline or an isotonic solution containing glucose and other supplementary agents (e.g., D-sorbitol, D-mannitol, sodium chloride etc.), and may be used in combination with suitable solubilizers such as alcohols (e.g., ethanol etc.), polyalcohols (e.g., propylene glycol, polyethylene glycol etc.) and nonionic surfactants (e.g., Polysorbate 80™, HCO-50 etc.). The oily solution includes sesame oil, soybean oil etc., and may be used in combination with solubilizer such as benzyl benzoate, benzyl alcohol etc.

Further, the above preventing or treating agent may be blended, for example, with the buffer (e.g., phosphate buffer. sodium acetate buffer etc.), soothing agents (e.g., benzalkonium chloride, procaine hydrochloride etc.), stabilizers (e.g., human serum albumin, polyethylene glycol etc.), preservatives (e.g., benzyl alcohol, phenol etc.), antioxidants etc. Usually, the prepared injection is introduced into suitable ampoules.

Since the thus obtained preparation is safe and low toxic, it can be administered, for example, to a human or a mammal (e.g., rat, mouse, rabbit, sheep, pig, cow, cat, dog, monkey, etc.).

A dose of said compound or a salt thereof is different depending on an administration subject, a subject organ, symptom and an administration method, and in the case of oral administration, the dose is usually about 0.1 to 100 mg, preferably about 1.0 to 50 mg and more preferably about 1.0 to 20 mg per day in a patient (60 kg) with arteriosclerosis. When parenterally administered, one time dose is different depending upon an administration subject, a subject organ, symptom and an administration method and, for example, in the form of an injection, it is advantageous to administer about 0.01 to 30 mg, preferably about 0.1 to 20 mg and more preferably about 0.1 to 10 mg per day through intravenous injection in a patient (60 kg) with arteriosclerosis. In the case of other animals, an amount calculated per 60 kg can be administered. (7) Quantification of the present receptor protein, a partial peptide thereof or a salt thereof

The present antibody can specifically recognize the present receptor protein and the like and is thus usable, for example, in quantification of the present receptor protein and the like in a test sample fluid, particularly in quantification thereof by sandwich immunoassays.

That is, the present invention provides:
(i) a method for quantifying the present receptor protein and the like in a test solution, which comprises allowing a test solution and the labeled receptor protein etc. to react competitively with the present antibody and determining the ratio of the present labeled receptor protein etc. bound to said antibody; and
(ii) a method of quantifying the present receptor protein and the like in a test solution, which comprises allowing a test solution to react with the present antibody immobilized on a carrier and the present labeled antibody simultaneously or successively and then measuring the activity of the labeling agent on the insolubilizing carrier.

In the above (ii), it is preferable that one of the two antibodies is an antibody recognizing an N-terminal region of the present receptor protein and the like, and the other antibody is an antibody reacting with a C-terminal region of the present receptor protein and the like.

The monoclonal antibody against the present receptor protein and the like (hereinafter, also referred to as the present monoclonal antibody) can be used not only for quantifying the present receptor protein and the like but also for detection thereof by tissue staining etc. For these purposes, the antibody molecule itself may be used, or an F (ab')₂, Fab' or Fab fraction of the antibody molecule may be used. The measurement method using the antibody against the present receptor protein and the like is not particularly limited as long as the method comprises detecting the amount of the antibody, the antigen or an antigen-antibody conjugate, corresponding to the amount of the antigen (e.g., the amount of the receptor protein) in a test solution, by chemical or physical means and calculating it on the basis of a standard curve prepared using standard solutions containing known amounts of the antigen. For example, nephelometry, the competitive method, the immunometric method and the sandwich method are preferably used, but the sandwich methoddescribedlaterisparticularlypreferablyusedinrespect of sensitivity and specificity.

The labeling agent used in the measurement method using a labeled substance includes, for example, a radioisotope, enzyme, fluorescent material and luminescent material. The radioisotope includes, for example, [¹²⁵I], [¹³¹I], [³H], [¹⁴C] etc. The enzyme described above is preferably a stable enzyme with high specific activity, which includes, for example, β-galactosidase, β-glucosidase, alkaline phosphatase, peroxidase and malate dehydrogenase. The fluorescent material includes, for example, fluorescamine and fluorescein isocyanate. The luminescent material includes,for example, luminol, luminol derivatives, luciferin and lucigenin. Further, abiotin-avidin system can also be used for binding the labeling agent with the antibody or antigen.

The antigen or antibody may be insolubilized by physical adsorption or via chemical bonding used conventionally for insolubilization or immobilization of proteins, enzymes etc. The carrier includes, for example, insoluble polysaccharides such as agarose, dextran and cellulose, synthetic resin such as polystyrene, polyacrylamide and silicon, and glass etc.

In the sandwich method, a test sample is allowed to react with the insolubilized present monoclonal antibody (primary reaction), then the labeled present monoclonal antibody is allowed to react therewith (secondary reaction), and the activity of the labeling agent on the insolubilizing carrier is measured, whereby the present receptor protein in the test sample fluid can be quantitatively determined. The primary and secondary reactions may be carried out in the reverse order, simultaneously, or separately after a given time. The labeling agent and the method of insolubilization may be in accordance with those described above.

The antibody used as the solid-phase antibody or as the labeling antibody in immunoassays by the sandwich method may not necessarily be one kind of antibody and may use a mixture of two or more kinds of antibodies for the purpose of improving measurement sensitivity etc.

In the method for measuring the present receptor protein and the like by the sandwich method according to the present invention, the present monoclonal antibodies used in the primary and secondary reactions are preferably those antibodies to which the receptor protein etc. are bound at different sites. That is, it is preferable that when the antibody used in, for example, the secondary reaction recognizes a C-terminal region of the receptor protein etc., the other antibody used in the primary reaction recognizes, for example, an N-terminal region other than the C-terminal region.

The present monoclonal antibody can be used not only in the sandwich method but also in other measurement systems such as competitive method, immunometric method and nephelometry. In the competitive method, an antigen in a test sample fluid and its labeled antigen are allowed to react competitively with the antibody, then the unreacted labeled antigen (F) and the labeled antigen (B) bound to the antibody are separated from each other (B/F separation), and the amount of either labeled B or F is determined to quantify the amount of the antigen in the sample. This reaction method employs either a liquid-phase method in which a soluble antibody is used as the antibody and polyethylene glycol and a second antibody against the above antibody are used in B/F separation, or a solid-phase method in which a solid-phase (i.e. immobilized) antibody is used as the first antibody, or a soluble antibody is used as the first antibody while a solid-phase antibody is used as the second antibody.

In the immunometric method, the antigen in a test solution and the solid-phase antigen are allowed to react competitively with a given amount of the labeled antibody, followed by separating the solid phase from the liquid phase, or alternatively the antigen in a test solution is allowed to react with an excess of the labeled antibody, and then the solid-phase antigen is added thereto to permit the unreacted labeled antibody to be bound to the solid phase, followed by separating the solid phase from the liquid phase. Then, the amount of the label in either phase is measured to quantify the amount of the antigen in the test solution.

Further, in nephelometry, the amount of insoluble precipitates in gel or solution, formed by antigen-antibody reaction, is measured. Laser nephelometry using laser scattering can be applied preferably to the case where precipitates are obtained in a small amount because of a very small amount of antigen in a test solution.

For application of these immunoassays to the measurement method of the present invention, it is not necessary to establish special conditions, procedures etc. A measurement system for the present receptor protein and the like or a salt thereof can be established in an ordinary manner by those skilled in the art by modifying the conventional conditions and procedures. The details of these general technical means can be referred to in general remarks, books etc. [for example, reference can be made of "Radioimmunoassays" edited by Hirhoshi Irie (published by Kodansha in 1974), "Radioimmunoassays" (second series) edited by Hiroshi Irie (published by Kodansha in 1979), "Enzyme Immunoassays" edited by Eiji Ishikawa et al. (published by Igakushoin in 1978), "Enzyme Immunoassays" (2nd edition) edited by Eiji Ishikawa et al. (published by Igakushoin in 1982), "Enzyme Immunoassays" (3rd edition) edited by Eiji Ishikawa et al. (published by Igakushoin in 1987), Methods in Enzymology, Vol. 70 (Immunochemical Techniques (Part A), Methods in Enzymology, Vol. 73 (Immunochemical Techniques (Part B)), Methods in Enzymology, Vol. 74 (Immunochemical Techniques (Part C)), Methods in Enzymology, Vol. 84 (Immunochemical Techniques (Part D: Selected Immunoassays)), and Methods in Enzymology, Vol. 92 (Immunochemical Techniques (Part E: Monoclonal Antibodies and General Immunoassay Methods)), Methods in Enzymology, Vol. 121 (Immunochemical Techniques (Part I: Hybridoma Technology and Monoclonal Antibodies)) (which are published by Academic Press Ltd.).

By using the present antibody in the manner as described above, the present receptor protein or a salt thereof can be quantified with good sensitivity.

Further, by quantifying the present receptor protein or a salt thereof in vivo by means of the present antibody, diagnosis of those diseases associated with the dysfunction of the present receptor protein can be performed.

Further, the present antibody can be used for specifically detecting the present receptor protein and the like in humor and tissues. In addition, the present antibody can be used for preparation of an antibody column used in purification of the present receptor protein and the like, for detection of the present receptor protein and the like in each fraction during purification, and for analysis of the behavior of the present receptor protein and the like in cells examined. (8) Neutralization by an antibody against the present receptor protein, a partial peptide thereof or a salt thereof

The activity of neutralizing the present receptor protein and the like by the antibody against the present receptor protein, a partial peptide thereof or a salt thereof means the inactivating activity of the signal transduction function in which the receptor protein participates. Therefore, the antibody having the neutralization activity can inactivate the signal transduction function in which the receptor protein participates, for example, the cell stimulating activity via the receptor protein (for example, the activity promoting or inhibiting arachidonic acid release, acetylcholine release, intracellular Ca²⁺ release, intracellular cAMP production, intracellular cGMP production, inositol phosphate production, cell membrane potential fluctuation, intracellular protein phosphorylation, c-fos activation, and reduction of pH). Therefore, it can be used for preventing and/or treating diseases attributable to excessive expression of the receptor protein. (9) Preparation of an animal having a DNA encoding the present G protein coupled receptor protein

By using the present DNA, transgenic animals expressing the present receptor protein and the like can be prepared. As the animal, mammals (for example, rat, mouse, rabbit, sheep, pig, cow, cat, dog, monkey etc.) (hereinafter abbreviated as animal in some cases), and particularly a mouse, rat etc. are preferable.

For transferring the present DNA to the intended animals, the DNA is generally advantageously used as a gene construct having the DNA bound to a region downstream from a promoter capable of expressing the DNA in animal cells. For example, in the case where the human-derived DNA of the present invention is to be transferred, a gene construct having the present DNA bound to a region downstream from a promoter derived an animal having high homology with the human-derived DNA and permitting expression of the present DNA in an animal is microinjected into for example mouse fertilized eggs, whereby DNA-transferred animals highly expressing the present receptor protein and the like can be prepared. As the promoter, for example, a virus-derived promoter, a ubiquitous expression promotor such as metallothionein, etc. can also be used, but preferably an NGF gene promoter and an enolase gene promoter etc. for specific expression in the brain are used.

The present DNA is transferred to cells at the stage of fertilized egg cells so as to secure the presence of the DNA in all embryonic cells and somatic cells of the intended mammals. The presence of the present receptor protein and the like in the embryonic cells in the prepared animals after transfer of the DNA means that the present receptor protein and the like are maintained in all embryonic cells and somatic cells in the offspring of the prepared animals . The offspring of the prepared animals, which has inherited said gene, has the present receptor protein and the like in all embryonic cells and somatic cells thereof.

After it is confirmed that the DNA-transferred animals of the present invention maintain the gene stably through crossbreeding, they can be bred generation after generation as the DNA-possesing animals under normal breeding environments. Further, male and female animals having the desired DNA are crossbred, whereby homozygote animals having the introduced gene in both homogenous chromosomes are obtained, and these male and female animals are crossbred, whereby all offspring can be bred generation after generation so as to have said DNA.

The animals having the present DNA transferred thereto are useful as animals for screening an agonist or antagonist for the present receptor protein and the like since the present receptor protein and the like are highly expressed in them.

The DNA-transferred animals of the present invention can also be used as a cell source for tissue culture. For example, by directly analyzing the DNA or RNA in tissues of the DNA-transferred mouse of the present invention or by analyzing tissues where the present receptor protein expressed by the gene is present, the present receptor protein and the like can be analyzed. Cells from tissues having the present receptor protein and the like are cultured in standard culture tissue techniques and these can be used to study, for example, the functions of cells from tissues (for example, those derived from cerebral or peripheral tissues) generally difficult to culture. Further, by using these cells, a pharmaceutical composition enhancing the functions of various tissues can also be selected. In addition, if there is a high-expression cell line, the present receptor protein and the like can be isolated and purified therefrom.

When bases or amino acids are expressed in abbreviations in the present specification and drawings, the following abbreviations in accordance with IUPAC-IUB Commission on Biochemical Nomenclature or based on customary abbreviations in this field are used. If amino acids can occur as optical isomers, L-isomers are referred to unless otherwise specified.
- DNA:: deoxyribonucleic acid
- cDNA:: complementary deoxyribonucleic acid
- A:: adenine
- T:: thymine
- G:: guanine
- C:: cytosine
- RNA:: ribonucleic acid
- mRNA:: messenger ribonucleic acid
- dATP:: deoxyadenosine triphosphate
- dTTP:: deoxythymidine triphosphate
- dGTP:: deoxyguanosine triphosphate
- dCTP:: deoxycytidine triphosphate
- ATP:: adenosine triphosphate
- EDTA:: ethylene diamine tetraacetate
- SDS:: sodium dodecyl sulfate
- Gly:: glycine

- Ala:: alanine
- Val:: valine
- Leu:: leucine
- Ile:: isoleucine
- Ser:: serine
- Thr:: threonine
- Cys:: cysteine
- Met:: methionine
- Glu:: glutamic acid
- Asp:: aspartic acid
- Lys:: lysine
- Arg:: arginine
- His:: histidine
- Phe:: phenyl alanine
- Tyr:: tyrosine
- Trp:: tryptophan
- Pro:: proline
- Asn:: asparagine
- Gln:: glutamine
- pGlu:: pyroglutamic acid
- Me:: methyl group
- Et:: ethyl group
- Bu:: butyl group
- Ph:: phenyl group
- TC:: thiazolidine-4 (R)-carboxamide group

The substituent groups, protecting groups and reagents appearing frequently in the present specification are expressed in the following symbols.
- Tos:: p-toluene sulfonyl
- CHO:: formyl
- Bzl:: benzyl
- Cl₂-Bzl:: 2,6-dichlorobenzyl
- Bom:: benzyloxymethyl
- Z:: benzyloxycarbonyl
- Cl-Z:: 2-chlorobenzyloxycarbonyl

- Br-Z:: 2-bromobenzyloxycarbonyl
- Boc:: t-butoxycarbonyl
- DNP:: dinitrophenol
- Trt:: trityl
- Bum:: t-butoxymethyl
- Fmoc:: N-9-fluorenyl methoxycarbonyl
- HOBt:: 1-hydroxybenztriazole
- HOOBt:: 3,4-dihydro-3-hydroxy-4-oxo-1,2,3-benzotriazine
- HONB:: 1-hydroxy-5-norbornane-2,3-dicarboxyimide
- DCC:: N,N'-dicyclohexyl carbodiimide

The sequence numbers in the Sequence Listing in the present specification show the following sequences:
SEQ ID NO: 1 shows an amino acid sequence of the novel mouse spleen-derived G protein coupled receptor protein mAL7T024 of the present invention.
SEQ ID NO: 2 shows a base sequence of a cDNA encoding thenovelmousespleen-derivedGproteincoupledreceptorprotein mAL7T024 of the present invention having the amino acid sequence represented by SEQ ID NO: 1.
SEQ ID NO: 3 shows a base sequence of primer 1 used for cloning a cDNA encoding the novel mouse spleen-derived G protein coupled receptor protein mAL7T024 of the present invention.
SEQ ID NO: 4 shows a base sequence of primer 2 used for cloning a cDNA encoding the novel mouse spleen-derived G protein coupled receptor protein mAL7T024 of the present invention.
SEQ ID NO: 5 shows an amino acid sequence of the novel human lung-derived G protein coupled receptor protein hAL7T024 of the present invention.
SEQ ID NO: 6 shows a base sequence of a cDNA encoding the novel human lung-derived G protein coupled receptor protein hAL7T024 of the present invention having the amino acid sequence represented by SEQ ID NO: 5.
SEQ ID NO: 7 shows a base sequence of primer 3 used for cloning a cDNA encoding the novel human lung-derived G protein coupled receptor protein hAL7T024 of the present invention.
SEQ ID NO: 8 shows a base sequence of primer 4 used for cloning a cDNA encoding the novel human lung-derived G protein coupled receptor protein hAL7T024 of the present invention.

The transformant *Escherichia coli* TOP10F'/pCR3.1-mAL024 obtained in Example 1 below has been deposited under FERM BP-6591 from December 2, 1998 with the National Institute of Bioscience and Human-Technology (NIBH), Agency of Industrial Science & Technology, Ministry of International Trade & Industry, and under IFO 16222 with Institute for Fermentation, Osaka, Japan (IFO) from November 25, 1998.

Hereinafter, the present invention is described in more detail by reference to the Reference Example and the Examples, which however are not intended to limit the scope of the invention. The genetic manipulation using *Escherichia coli* was conducted according to methods described in Molecular Cloning.

### Example 1

Cloning and nucletide sequencing of a cDNA encoding a mouse spleen G protein coupled receptor protein

PCR reaction was performed using a mouse spleen-derived cDNA as a template and two primers, that is, primer 1 (SEQ ID NO: 3) and primer 2 (SEQ ID NO: 4). The reaction solution in the reaction was composed of 1/10 volume of the above cDNA as a template, 1/50 volume of Advantage cDNA Polymerase Mix (Clontech), 0.2 µM each of primer 1 (SEQ ID NO: 3) and primer 2 (SEQ ID NO: 4) and 200 µM dNTPs, and the volume of the solution was adjusted to 25 µl by adding a buffer attached to the enzyme. In the PCR reaction, after (1) a cycle at 94 °C for 30 seconds, (2) a cycle at 94 °C for 5 seconds and at 68 °C for 2 minutes was repeated 35 times, and finally (3) elongation reaction at 68 °C for 7 minutes was performed. After the PCR reaction, the reaction product was subcloned into plasmid vector pCR3.1 (*In vitrogen*) according to the protocol of a TA cloning kit (*In vitrogen).* The resulting vector was introduced into *Escehrichia coli* TOP10F', and clones having the cDNA were selected in an LB agar medium containing ampicillin, and individual clones were examined for their sequence, and as a result, a cDNA sequence (SEQ ID NO: 2) encoding a novel G protein coupled receptor protein was obtained. The novel G protein coupled receptor protein comprising an amino acid sequence (SEQ ID NO: 1) derived from this cDNA was designated mAL7T024, and its transformant was designated *Escherichia coli* TOP10F'/pCR3.1-mAL024.

### Example 2

Cloning and nucleotide sequencing of a cDNA for the human lung-derived novel G protein coupled receptor protein hAL7T024

PCR reaction was performed using a human lung cDNA (Clontech) as a template and two primers, that is, primer 3 (SEQ ID NO: 7) and primer 4 (SEQ ID NO: 8). The reaction solution in the reaction was composed of 1/25 volume of the above cDNA as a template, 1/50 volume of Advantage cDNA Polymerase Mix (Clontech), 0.2 µM each of primer 3 (SEQ ID NO: 7) and primer 4 (SEQ ID NO: 8) and 200 µM dNTPs, and the volume of the solution was adjusted to 25 µl by adding a buffer attached to the enzyme. In the PCR reaction, after (1) a cycle at 94 °C for 30 seconds, (2) a cycle at 94 °C for 5 seconds and at 68 °C for 2 minutes was repeated 35 times, and finally (3) elongation reaction at 68°C for 7 minutes was performed. After the PCR reaction, the reaction product was subcloned into plasmid vector pCR3.1 (*In vitrogen*) according to the protocol of a TA cloning kit (*In vitrogen*). The resulting vector was introduced into *Escherichia coli* DH5α, and clones having the cDNA were selected in an LB agar medium containing ampicillin, and individual clones were examined for their sequence, and as a result, a transformant (*Escherichia coli* DH5α/pCR3.1-h024) harboring a cDNA sequence (SEQ ID NO: 6) encoding a novel G protein coupled receptor protein was obtained. The novel G protein coupled receptor protein comprising an amino acid sequence (SEQ ID NO: 5) derived from this cDMA was designated hAL7T024.

### Industrial Applicability

The G protein coupled receptor protein of the present invention, a partial peptide thereof or a salt thereof, as well as a polynucleotide encoding the same (for example, DNA, RNA and a derivative thereof), is useful in (1) a method for determining a ligand (agonist), (2) acquisition of an antibody and antiserum thereto, (3) construction of an expression system for a recombinant receptor protein thereof, (4) development of a binding assay system using said expression system and screening of a pharmaceutical candidate compound, (5) practice of a drug design based on comparison with structurally analogous ligand receptors, (6) reagents for preparation of probes and PCR primers in gene diagnosis, (7) preparation of a transgenic animals or (8) a pharmaceutical composition for a preventing and treating genetic agent etc.

## Claims

1. A G protein coupled receptor protein which comprises an amino acid sequence identical or substantially identical to an amino acid sequence represented by SEQ ID NO: 1 or a salt thereof.

2. The G protein coupled receptor protein according to claim 1, wherein the substantially identical amino acid sequence is an amino acid sequence represented by SEQ ID NO: 5.

3. Apartial peptide of the Gprotein coupled receptor protein according to claim 1 or a salt thereof.

4. A polynucleotide which comprises a polynucleotide having a base sequence encoding the G protein coupled receptor protein according to claim 1.

5. The polynucleotide according to claim 4, which is a DNA.

6. The polynucleotide according to claim 4, which has a base sequence represented by SEQ ID NO: 2 or SEQ ID NO: 6.

7. A recombinant vector which comprises the polynucleotide according to claim 4.

8. A transformant transformed with the recombinant vector according to claim 7.

9. A method for manufacturing the G protein coupled receptor protein according to claim 1 or a salt thereof, which comprises culturing the transformant according to claim 8 to produce the G protein coupled receptor protein according to claim 1.

10. An antibody to the G protein coupled receptor protein according to claim 1 or the partial peptide according to claim 3 or a salt thereof.

11. The antibody according to claim 10 , which is a neutralizing antibody which inactivates signal transduction of the G protein coupled receptor protein according to claim 1.

12. A diagnostic agent which comprises the antibody according to claim 10.

13. A ligand for the G protein coupled receptor protein according to claim 1 or a salt thereof, which is obtained by using the G protein coupled receptor protein according to claim 1 or the partial peptide according to claim 3 or a salt thereof.

14. A pharmaceutical composition which comprises the ligand according to claim 13.

15. A method for determining a ligand for the G protein coupled receptor protein according to claim 1 or a salt thereof, which comprises using the G protein coupled receptor protein according to claim 1 or the partial peptide according to claim 3 or a salt thereof.

16. A method for screening a compound or a salt thereof which alters binding property of a ligand with the G protein coupled receptor protein or a salt thereof according to claim 1, which comprises using the G protein coupled receptor protein according to claim 1 or the partial peptide according to claim 3 or a salt thereof.

17. A kit for screening a compound or a salt thereof which alters binding property of a ligand with the G protein coupled receptor protein or a salt thereof according to claim 1, which comprises the G protein coupled receptor protein according to claim 1 or the partial peptide according to claim 3 or a salt thereof.

18. A compound or a salt thereof which alters binding property of a ligand with the G protein coupled receptor protein or a salt thereof according to claim 1, which is obtained by using the method for screening according to claim 16 or the kit for screening according to claim 17.

19. A pharmaceutical composition which comprises a compound or a salt thereof which alters binding property of a ligand with the G protein coupled receptor protein or a salt thereof according to claim 1, which is obtained by the method for screening according to claim 16 or the kit for screening according to claim 17.

20. A polynucleotide which hybridizes with the polynucleotide according to claim 4 under highly stringent conditions.

21. A polynucleotide which comprises a base sequence complementary to the polynucleotide according to claim 4 or a part thereof.

22. A method for quantifying an mRNA for the G protein coupled receptor protein according to claim 1, which comprises using the polynucleotide according to claim 4 or a part thereof.

23. A method for quantifying the G protein coupled receptor protein according to claim 1, which comprises using the antibody according to claim 10.

24. A diagnistic agent for diseases associated with the function of the G protein coupled receptor according to claim 1, which comprises using the quantifying method according to claim 22 or claim 23.

25. A method for screening a compound or a salt thereof which alters expression of the G protein coupled receptor according to claim 1 to be expressed, which comprises using the quantifying method according to claim 22 or claim 23.

26. A compound or a salt thereof which alters expression of the G protein coupled receptor protein according to claim 1, which is obtained by the method for screening according to claim 25.
